# EUROPÄISCHE PATENTANMELDUNG

(11) **EP 1 906 304 A2**
(43) Veröffentlichungstag der Anmeldung: **02.04.2008**
(21) Anmeldenummer: 07111954.9
(22) Anmeldetag: 06.07.2007
(51) Int. Cl.: G06F 9/44

(54) **System zur Erzeugung und zum Betrieb einer Softwareapplikation für medizinische Bildgebung**

(30) Priorität: 29.09.2006 DE 102006046310
(71) Anmelder: SIEMENS AKTIENGESELLSCHAFT, 80333 München (DE)
(72) Erfinder: Dorn, Karlheinz, 90562, Kalchreuth (DE); Hewett, Andrew John, 91058, Erlangen (DE); Becker, Detlef, 91096, Möhrendorf (DE); Spitzner, Christian, 97526, Sennfeld (DE); Ax, Antonius, 91090, Effeltrich (DE); Dürbeck, Norbert, 91180, Heideck (DE); Hirschbeck, Frank, 91054, Erlangen (DE); Krotz, Dieter, 91056, Erlangen (DE); Michel, Armin, 91058, Erlangen (DE); Pusztai, Artur, 91054, Erlangen (DE); Sinha, Subrata, 91054, Erlangen (DE)

(57) **Zusammenfassung**

Es wird ein effektives und einfach handhabbares System (1) zur Erzeugung und zum Betrieb einer Softwareapplikation für medizinische Bildgebung, angegeben, mit mindestens einem Framework (2,22,34,45,58,69,81), das eine Serviceschicht (3) sowie eine dieser als Applikations-Programmier-Schnittstelle übergeordnete Toolkit-Schicht (4) aufweist, wobei Funktionen der Toolkit-Schicht (4) und der Serviceschicht (3) in jeweils einer Anzahl von Komponenten zusammengefasst sind, die streng hierarchisch angeordnet sind derart, dass auf eine beliebige Komponente stets ausschließlich von einer übergeordneten Komponente aus zugegriffen werden kann.

## Beschreibung

Die Erfindung bezieht sich auf ein System zur Erzeugung und zum Betrieb einer Softwareapplikation für medizinische Informationsverarbeitung, insbesondere in der medizinischen Bildgebung.

Softwareapplikationen werden im medizinischen Bereich, insbesondere im Bereich der (digitalen) medizinischen Bildgebung eingesetzt, um medizinische Daten wie z.B. digitale Untersuchungsbilder, Patientendaten, Befundungsberichte, etc. zu erstellen, zu modifizieren, zwischen verschiedenen Knoten eines medizinischen Netzwerkes zu übertragen und zu archivieren. Als medizinisches Netzwerk wird hierbei ein Ensemble von Geräten bezeichnet, die für den Zweck der medizinischen Bildgebung zum Datenaustausch miteinander verbunden sind. Die Geräte eines solchen medizinischen Netzwerkes umfassen insbesondere medizinische Modalitäten, Befundungsrechner, Server, Datenbanken, und sonstige Datenverarbeitungsanlagen. Als medizinisches Netzwerk wird insbesondere ein sogenanntes DICOM-Netz bezeichnet, bei dem die Geräte (DICOM-Knoten) auf Basis des DICOM-Standards miteinander kommunizieren. Als Modalität oder Bildquelle wird in diesem Sinne ein bilderzeugendes medizinisches Untersuchungsgerät bezeichnet, insbesondere ein Computertomograph, Magnetresonanztomograph, Ultraschallscanner, etc.

Ein abgegrenzter funktionell-thematischer Bereich wie der der medizinischen Bildgebung - in der Softwareentwicklung auch als "Domain" bezeichnet - erfordert speziell angepasste Softwarelösungen. Gerade in der Domain der medizinischen Bildgebung nehmen aufgrund der Größe heutiger medizinischer Einrichtungen, der Vielfalt der zu bewältigenden Aufgaben und der zunehmenden elektronischen Vernetzung verschiedener medizinischer Teilbereiche die hierfür domain-spezifisch konzipierten Softwareapplikationen einen erheblichen und stetig zunehmenden Grad an Komplexität an.

Die Erstellung einer derartigen Softwareapplikation erfordert von einem Entwickler ein hohes Maß an programmiertechnischen Wissen und Erfahrung. Bisher werden Softwareapplikationen für die Domain der medizinischen Bildgebung nahezu ausschließlich monolithisch, d.h. als ein Applikationsblock erstellt. Ein derartiger Applikationsblock muss bei notwendigen Änderungen, wie z.B. bei einem Software-Update, immer in Gänze geändert werden. Dies führt zu einem immensen Aufwand bei der Erstellung der Applikation und deren Wartung und erschwert somit eine zeitgemäße Weiterentwicklung. Darüber hinaus ist eine monolithische Softwareapplikation in der Regel relativ fehleranfällig.

Softwareapplikationen für die medizinische Bildgebung weisen einerseits üblicherweise eine komplexe Softwarearchitektur mit mehreren Schichtebenen auf. Andererseits zerfallen die Funktionen und Dienste einer solchen Softwareapplikation in mehrere funktionale Untergruppen, die im Folgenden als Sub-Domains bezeichnet werden. Für den Bereich der medizinischen Bildgebung sind insbesondere die Sub-Domains Datenmanagement, (Daten-)Transfer, Aufgabenlistenmanagement (worklist management), Bildbearbeitung (image processing), Volumenbearbeitung (volume processing), Berichterstellung (reporting) und Bilderfassung bzw. Untersuchung (examination) von prominenter Bedeutung.

Die Sub-Domain "Datenmanagement" umfasst die Gesamtheit der auf den Datenzugriff bezogenen Funktionalität einer gattungsgemäßen Softwareapplikation, insbesondere fundamentale Funktionen wie das Lesen von Daten aus einem Dateisystem, das Schreiben von Daten in ein solches und das Löschen von gespeicherten Daten sowie das Erzeugen von Dateien. Als "Dateisystem" ist hierbei eine Struktur bezeichnet, in der Daten in Form von Dateien gespeichert sind, und die ein Ordnungs- und Zugriffssystem für diese Daten bildet. Ein Dateisystem kann einem dabei physischen Speichermedium, z.B. einer Festplatte, CDrom, etc. entsprechen, oder als rein logische Struktur, z.B. als virtuelles Laufwerk, ausgebildet sein. Der Begriff Datenmanagement umfasst des weiteren hierauf aufbauende Funktionalitäten, insbesondere Funktionen zur Suche und Identifizierung von bestimmten Daten, Zugangsregelungen zu den hinterlegten Daten, lokale Zwischenspeicherung (caching) von Daten. Aufgrund der Besonderheit der im Bereich der medizinischen Bildgebung verarbeiteten Daten, insbesondere medizinischer Bilddaten, die sich durch sehr hohen Speicherbedarf und eine enge inhaltliche Verknüpfung von Bildinformation und zugehöriger Textinformation, sogenannter Meta-Daten, auszeichnen, unterliegt auch das im Bereich der medizinischen Bildgebung eingesetzte Datenmanagement domain-spezifischen Anforderungen. Zumal die Daten in der modernen medizinischen Bildgebung üblicherweise durch eine Vielzahl von Benutzern an einer Vielzahl von Knoten eines Computernetzwerkes bearbeitet werden, ist gerade dort ein effektives Datenmanagement von besonderer Bedeutung, um eine sichere und effektive Funktion einer für diesen Bereich konzipierten Softwareapplikation sicherzustellen. Der Begriff Datenmanagement ist nachfolgend als "DM" abgekürzt.

Die Sub-Domain (Daten-)"Transfer" (nachfolgend abgekürzt als "TF") umfasst die Gesamtheit der Funktionalität einer gattungsgemäßen Softwareapplikation, die auf die Übertragung von Daten, insbesondere medizinischen Bild-, Volumen- und Text-und Workflowdaten zwischen zwei Geräten oder Dateisystemen eines medizinischen Netzwerkes, z.B. im Rahmen des DICOM-Standards, ausgerichtet ist.

Die Sub-Domain "Aufgabenlisten(-management)" bzw. "Worklist (-management)" (nachfolgend abgekürzt als "WL") umfasst die Gesamtheit der Funktionalität einer gattungsgemäßen Softwareapplikation, die auf die Erstellung und Kontrolle sogenannter Aufgabenlisten (oder worklists) gerichtet ist. Solche Aufgabenlisten werden im Bereich der medizinischen Bildgebung insbesondere verwendet, um Schritte eines arbeitsteilig organisierten medizinischen Arbeitsablaufs (workflow) den einzelnen Knoten des medizinischen Netzwerkes zuzuweisen und die Abarbeitung des Arbeitsablaufs automatisiert zu koordinieren. Solche Aufgabenlisten sind insbesondere im Rahmen des DICOM-Standards für den Bereich der medizinischen Bildgebung standardisiert.

Die Sub-Domain "Bildbearbeitung" bzw. "image processing" (nachfolgend abgekürzt als "IP") beinhaltet die Gesamtheit der Funktionalität einer gattungsgemäßen Softwareapplikation, die auf die Modifikation und Anzeige von (zweidimensionalen) medizinischen Bilddaten gerichtet ist, umfassend z.B. Kontrast- und Helligkeitsoperationen sowie sonstige Farbänderungsoperationen, Glättung, Scharfzeichnung oder sonstige Filter, 2D-Mustererkennung (Segmentierung), etc.

Die Sub-Domain "Volumenbearbeitung" (nachfolgend abgekürzt als "VOL") beinhaltet die Gesamtheit der Funktionalität einer gattungsgemäßen Softwareapplikation, die auf die Erstellung mehrdimensionaler Strukturen (3D-/4D-/...-Szenen) anhand von medizinischen Bilddaten sowie die Handhabung, Bearbeitung und Darstellung solcher Strukturen oder deren zweidimensionaler Schritte und Projektionen gerichtet ist. Volumenbearbeitung umfasst insbesondere die visuell-räumliche Darstellung dreidimensionaler Strukturen (volume rendering), 3D-Animation, die Erzeugung von Schnittbildern, etc. Als "Volumendaten" werden im Folgenden Daten verstanden, die eine Bild- oder Strukturinformation in Abhängigkeit eines mehr als zweidimensionalen Koordinatensystems beinhalten. Im Unterschied hierzu wird von "Bilddaten" nur in Hinblick auf Daten gesprochen, die eine zweidimensionale Bild- oder Strukturinformation beinhalten. Als Bilddaten werden hierbei neben herkömmlichen Bildern im weiteren Sinne auch andere Daten mit zweidimensionalem Charakter, insbesondere Messkurven wie z.B. sogenannte Wave-Forms oder Spektraldaten, bezeichnet. Die Unterscheidung zwischen Bilddaten und Volumendaten ist insofern wichtig, als Bilddaten im Gegensatz zu Volumendaten direkt auf einem Bildschirm oder Ausdruck darstellbar sind, und somit hinsichtlich der jeweiligen Datenbearbeitung gänzlich anderen Anforderungen unterliegen.

Die Sub-Domain "Berichterstellung" bzw. "reporting" (nachfolgend abgekürzt als REP) umfasst die Gesamtheit der Funktionalität einer gattungsgemäßen Softwareapplikation, die die Erstellung medizinischer Berichte, insbesondere Befundungsberichte und Präsentationen, insbesondere unter Nutzung strukturierter Daten (z.B. nach dem DICOM- oder HL7-Standard) unterstützt.

Die Sub-Domain "Bilderfassung" bzw. "Untersuchung / Examination" (nachfolgend abgekürzt als "EXAM") umfasst schließlich die Gesamtheit der Funktionalität einer gattungsgemäßen Softwareapplikation, die auf die Erstellung medizinischer Bilddaten, d.h. auf die Bilderzeugung von Untersuchungsergebnissen an der bildgebenden Modalität, inklusive der Echtzeit-Darstellung (Life-Display) der erfassten Bilddaten sowie sonstiger Dienste, die die Bildaufnahme unterstützen, gerichtet ist.

Bei herkömmlichen Applikationen für die Domain der medizinischen Bildgebung hängen die Komponenten einer Applikationsschicht in der Regel in komplexer Weise sowohl von Komponenten derselben Schicht als auch von Komponenten anderer Schichten ab, wobei infolge solcher Abhängigkeiten insbesondere häufig verschiedene Sub-Domains untrennbar miteinander verknüpft sind. Dies erschwert die Weiterentwicklung von Softwareapplikationen, zumal aufgrund des Abhängigkeitsgeflechts eine Änderung einer Komponente in der Regel eine Kaskade von Änderungen nach sich zieht. Die Weiterentwicklung einer solchen Softwareapplikation ist auch äußerst fehleranfällig, zumal bereits eine bei einer Änderung übersehene Abhängigkeit die Stabilität der Softwareapplikation in beträchtlichem Maße beinträchtigen kann.

Zur Vereinfachung der Applikationsentwicklung gibt es so genannte Frameworks als Unterstützungsumgebungen für einen Applikationsentwickler. Ein Framework kapselt häufig die einzelnen Schichten einer Softwareapplikation innerhalb einer generischen Laufzeitumgebung und ermöglicht damit einen plattform- und/oder Ablaufort-unabhängigen Ablauf der Applikation.

Ein wichtiges Framework ist das .NET Framework der Firma Microsoft Corporation. Dieses Framework bietet die Möglichkeit, unterschiedlichste Programmiersprachen, wie C#, Visual Basic.NET, C++/CLI oder JScript.NET, als Grundlage für die Programmierung einer n-schichtigen Applikation zu verwenden. Unabhängig von der Art der verwendeten Programmiersprache wird die Applikation und/oder jeweilige Architekturschicht der Applikation in eine "Zwischensprache" ("Common Intermediate Language", kurz CIL, früher auch als "Microsoft Intermediate Language", abgekürzt MSIL, bezeichnet) umgewandelt.

Große Bedeutung haben dabei die zwischen den einzelnen Schichten der Applikation notwendigen Anwendungsschnittstellen (engl.: application programing interface; kurz API). Man unterscheidet zwischen funktionsorientierten, interfaceorientierten und protokollorientierten Anwendungsschnittstellen. Im Gegensatz zu funktionsorientierten und interfaceorientierten Anwendungsschnittstellen sind protokollorientierte Anwendungsschnittstellen unabhängig von dem Betriebssystem der Plattform und der Art der zu verbindenden Schichten der Applikation.

Der Erfindung liegt vor diesem Hintergrund die Aufgabe zugrunde, ein für den Einsatz in der Domain der medizinischen Bildgebung geeignetes System zur Erzeugung und zum Betrieb einer Softwareapplikation anzugeben, das eine vereinfachte Applikationserstellung sowie einen effektiven Betrieb einer solchen Applikation ermöglicht.

Diese Aufgabe wird erfindungsgemäß gelöst durch die Merkmale des Anspruchs 1.

Danach ist im Rahmen des Systems mindestens ein sub-domain-spezifisches Framework vorgesehen. Das oder jedes Framework umfasst mindestens zwei Schichten, nämlich eine Service-schicht und eine dieser übergeordnete Toolkit-Schicht. Die Toolkit-Schicht bildet dabei ein API und liefert somit "Werkzeuge" (oder "Tools"), mit denen ein Applikationsentwickler im Sinne einer schnellen Applikationsentwicklung (RAD - rapid applikation developement) vereinfacht auf die Funktionalität der Serviceschicht zugreifen kann. Die Toolkit-Schicht bildet somit die für den Applikationsentwickler "sichtbare Oberfläche" des Systems. Die Komponenten bzw. Softwaremodule und Funktionen der Serviceschicht selbst können dagegen für einen Applikationsentwickler "verborgen" sein. Auf die letzteren Komponenten und Funktionen muss der Applikationsentwickler also nicht direkt zugreifen (Ein Direktzugriff einer Applikation auf Komponenten und Funktionen der Service-Schicht kann ungeachtet dessen aber als Option zugelassen sein).

Zusätzlich zu der eigentlichen Serviceschicht ist optional eine dieser wiederum untergeordnete Web-Serviceschicht vorgesehen. In die Web-Serviceschicht sind dabei diejenigen Funktionalitäten des Systems ausgelagert, die von einem entfernten Knoten eines medizinischen Netzwerkes abrufbar sind, insbesondere zentrale Dienste, die von dem gesamten Netzwerk oder Teilen davon gemeinsam benutzt werden. Die Serviceschicht ist in dieser Ausführung des Systems derart ausgebildet, das sie auch ohne die Dienste der Web-Serviceschicht lauffähig ist und in diesem Fall ausschließlich auf lokal ausgeführbare Funktionalitäten zugreift. Durch die strenge Trennung von Serviceschicht und Web-Serviceschicht wird so auf einfache Weise ermöglicht, dass ein- und dieselbe Ausführung des Systems sowohl im Online-Betrieb (d.h. als Teil des medizinischen Netzwerks) als im Offline-Betrieb (d.h. auf einem isolierten Rechner) lauffähig ist.

Die Komponenten der Web-Serviceschicht teilen dessen ungeachtet die grundlegenden Eigenschaften der Servicekomponenten. Die nachfolgenden allgemeinen Ausführungen zu Komponenten der Serviceschicht sind daher auch auf Komponenten der Web-Serviceschicht zu beziehen.

Im Sinne der obigen und nachfolgenden Ausführungen wird der Begriff "Funktion" allgemein als Bezeichnung für einen Softwarebaustein verwendet, der eine bestimmte Funktionalität erfüllt. Der Begriff wird dagegen unabhängig von der konkreten programmtechnischen Realisierung dieser "Funktion" verwendet. Er umfasst somit insbesondere Funktionen im Sinne der klassischen prozeduralen Programmierung, Objekte im Sinne einer objektorientierten Programmierung, Plugins, Assemblys etc.

Die Begriffe "Komponente" und "(Software-)Modul" werden im Sinne der obigen und nachfolgenden Ausführungen synonym als Bezeichnung für eine zusammengefasste Gruppe von Funktionen gemäß obiger Definition verwendet. Hierbei kann es sich um ein selbstständig lauffähiges Programm bzw. Teilprogramm oder um eine Sammlung von für sich gesehen nicht lauffähigen Funktionen handeln. Der Begriff Komponente bzw. Modul umfasst in diesem Sinne insbesondere Funktions- und Klassenbibliotheken sowie Sammlungen von Plugins und/oder Assemblys od. dgl.

Komponenten der Serviceschicht werden nachfolgend als Servicekomponenten, Komponenten der Toolkit-Schicht als Toolkits bezeichnet.

Im Sinne ihrer jeweiligen Überordnung bzw. Unterordnung sind die Komponenten der Serviceschicht und Toolkit-Schicht sowohl innerhalb der Schichten als auch schichtübergreifend streng hierarchisch angeordnet. Die definierende Charakteristik dieser Hierarchie besteht dabei darin, dass von einer beliebigen Komponente aus ausschließlich Zugriffsmöglichkeiten auf hierarchisch untergeordnete Komponenten vorgesehen sind. Eine Komponente ist mit anderen Worten nur von untergeordneten Komponenten abhängig. Umgekehrt enthält keine Komponente eine Information über eine dieser neben - oder übergeordnete Komponente. Die hierarchische Ordnung der Komponenten geht einher mit einem zunehmenden Abstraktionsgrad und einer zunehmenden Vereinheitlichung der in den jeweiligen Komponenten enthaltenen Funktionen. Niederrangige Komponenten enthalten somit vergleichsweise grundlegende Funktionen, höherrangige Komponenten entsprechend Funktionen eines höheren Abstraktionsgrades, die bedarfsweise auf die Funktionen niederrangiger Komponenten zugreifen.

Durch die streng hierarchische Anordnung der Komponenten wird die Fortentwicklung des bzw. der Frameworks erheblich vereinfacht. Infolge der Hierarchie wird nämlich sichergestellt, dass eine Änderung einer beliebigen ersten Komponente weitere Änderungen ausschließlich in denjenigen Komponenten erforderlich macht, die dieser ersten Komponente entlang des Hierarchiepfads übergeordnet sind. Komponenten, die der besagten ersten Komponente nebengeordnet oder untergeordnet sind, sind dagegen von der Änderung zwangsweise unbeeinflusst. Durch die hierarchische Komponentenanordnung wird daneben auch eine erhebliche Vereinfachung der Softwarearchitektur, und damit eine deutlich verringerte Fehleranfälligkeit erzielt.

In bevorzugter Ausführung der Erfindung unterscheiden sich die Servicekomponenten von Toolkits grundlegend hinsichtlich ihrer programmiertechnischen und ablauftechnischen Eigenschaften.

Danach sind die Servicekomponenten einfach-instanziierbar und multi-thread-fähig ausgebildet, während Toolkits mehrfach-instanziierbar und single-thread-fähig ausgebildet sind.

Als "einfach-instanziierbar" wird eine Komponente bzw. eine Funktion dann bezeichnet, wenn sie im Rahmen einer Applikation lediglich in einer einzigen Verkörperung bzw. "Instanz" eingesetzt werden kann. Im Gegensatz dazu kann eine Komponente bzw. Funktion, die "mehrfach-instanziierbar" ausgebildet ist, innerhalb der Applikation in Form mehrerer Instanzen (der gleichen Softwareversion oder unterschiedlicher Softwareversionen) mehrfach nebeneinander eingesetzt werden.

Als "thread" wird ein Ablaufmuster oder Ausführungsstrang bezeichnet, gemäß dem die Befehlssequenzen eines Computerprogramms der Reihe nach abgearbeitet werden. Bei einer Komponente bzw. Funktion, die "single-thread-fähig" (singlethreaded) ausgebildet ist, existiert stets lediglich ein solcher "thread" oder Ausführungsstrang. Mehrere anstehende Aufgaben werden durch eine solche Komponente bzw. Funktion stets nacheinander abgearbeitet. Bei einer "multi-thread-fähig" (multi-threaded) ausgebildeten Komponente oder Funktion können dagegen mehrere "threads" unabhängig voneinander und gleichzeitig ausgeführt werden.

Erkanntermaßen ist eine multi-thread-fähig ausgebildete Komponente zwar wesentlich leistungsfähiger als eine single-thread-fähige Komponente, weil sie die Rechenleistung der zur Verfügung stehenden Hardware stets ausschöpfen, insbesondere alle vorhandenen Prozessoren einsetzen kann. Jedoch ist andererseits die Programmierung multi-thread-fähiger Prozesse wesentlich schwieriger und fehleranfälliger als eine Single-thread-Programmierung.

Indem Service- und Toolkits wie vorstehend beschrieben gegensätzlich ausgebildet sind, werden die jeweiligen Vorteile der beschriebenen Programmierungsvarianten synergetisch genutzt. So kommt ein Applikationsentwickler in der Regel nur mit den Toolkits direkt in Berührung und kommt somit in den Genuss der leichteren Handhabbarkeit der Single-thread-Programmierung. Die mehrfache Instanziierbarkeit dieser Komponenten ist dabei zweckmäßig, um insbesondere komplexen Applikationen genügend Anknüpfungsstellen an die Funktionen des Frameworks zur Verfügung stellen zu können.

Die Servicekomponenten sind dagegen dem direkten Zugriff des Applikationsentwicklers in der Regel entzogen, so dass die infolge der Multi-thread-Fähigkeit vergleichsweise schwierige Handhabung dieser Komponenten bei der Applikationsentwicklung keinen Nachteil darstellt. Dafür gewährleistet die Multi-Threadfähigkeit der Servicekomponenten - auf die bei der Ausführung der Applikation ein Großteil der eigentlichen Rechenleistung abfällt - eine hohe Leistungsfähigkeit der Applikation. Durch die Einfach-Instanziierbarkeit der Servicekomponenten wird gleichzeitig eine vergleichsweise einfach strukturierte Softwarearchitektur in der Serviceschicht erzielt.

Im Sinne einer schnellen Applikationsentwicklung (auch als rapid application development, kurz RAD bezeichnet) ist zweckmäßigerweise vorgesehen, dass Toolkits auf einer visuellen Programmierumgebung instanziierbar und über Methoden und Ereignisse verknüpfbar ist.

Für eine strukturierte und somit weiter vereinfachte Applikationsprogrammierung ist die Programmierumgebung vorteilhafterweise in mehrere Seiten gliederbar, auf denen die Toolkits instanziierbar und verknüpfbar sind. In bevorzugter Ausbildung ist dabei für das bzw. ein jedes Framework eine sogenannte Master-Seite erzeugbar, die eine Vorlage bzw. einen Rahmen für die frameworkspezifischen Funktionalität einer Applikation darstellt. Das oder jedes Framework weist dabei eine oder mehrere Toolkits auf, die dieser Master-Seite zuordenbar ist und die damit die Eigenschaften dieser Master-Seite bestimmen. Optional ist darüber hinaus für ein oder mehrere Frameworks die Möglichkeit vorgesehen, den durch die Master-Seite vorgegebenen Funktionalitätsrahmen durch mehrere gegeneinander austauschbare Inhalte auszugestalten und/oder zu erweitern. Hierzu können unter Nutzung der Master-Seite als Vorlage mehrere Inhalts-Seiten erzeugt werden. Im Rahmen der betreffenden Frameworks können weitere Toolkits (nachfolgend als "Baukastenmodule" bezeichnet) erzeugt werden, deren jede einer Inhalts-Seite zuordenbar ist, und die den konkreten Inhalt dieser Seite spezifiziert.

Bevorzugt umfasst das System eine mit der Toolkit-Schicht korrespondierende Präsentationsschicht. Die Präsentationsschicht stellt eine (insbesondere graphische) Benutzerschnittstelle zur Verfügung, enthält also Funktionen zur Anzeige von Daten sowie zur Abgabe von Steuerbefehlen. Innerhalb der Präsentationsschicht ist zu jeder Seite der Toolkit-Schicht eine korrespondierende Präsentationsseite vorgesehen. In der Regel (aber nicht zwingend) ist darüber hinaus zu jedem Toolkit eine korrespondierende Präsentationskomponente vorgesehen. Die oder jede Präsentationsseite korrespondiert mit der entsprechenden Seite der Toolkit-Schicht. Die korrespondierenden Seiten der Toolkit-Schicht und Präsentationsschicht wirken somit als Backend und Frontend einer Benutzeroberfläche einer Applikation zusammen, um Steuerbefehle eines Benutzers an die Serviceschicht zu übermitteln und im Gegenzug Ausgaben der Serviceschicht für den Benutzer anzuzeigen. Die Toolkit-Schicht und die Präsentationsschicht korrespondieren dabei zweckmäßigerweise über eine Verschaltungsschnittstelle miteinander, die flexibel für unterschiedliche Deployments, also Verteilungen der Präsentationsschicht und Toolkit-Schicht auf einen oder mehrere Knoten des medizinischen Netzwerkes konfigurierbar ist.

Wiederum im Sinne einer schnellen Applikationsentwicklung ist auch im Rahmen der Präsentationsschicht eine visuelle Programmierumgebung vorhanden, auf der die oder jede Präsentationskomponente instanziierbar und über Methoden und Ereignisse verknüpfbar sind.

Um das oder jedes Framework flexibel an die Anforderungen der konkreten Applikation anpassen und gegebenenfalls erweitern zu können, sind die Funktionen der Servicekomponenten, sowie optional auch der Toolkits und Präsentationskomponenten bevorzugt ganz oder zumindest teilweise in Form von Plugins realisiert. Die Plugins sind zweckmäßigerweise derart implementiert, dass sie zur Laufzeit der auf dem Framework aufbauenden Applikation eingebunden oder deaktiviert werden können.

Unabhängig von dem vorstehend eingeführten Schichtaufbau - also der Serviceschicht und der Toolkit-Schicht sowie, falls vorhanden, der Web-Serviceschicht und/oder der Präsentationsschicht - sind bezüglich des oder jedes Frameworks eine sogenannte Stabilitätsschicht und eine sogenannte Entwicklungsschicht definiert, wobei die Stabilitätsschicht einen hierarchisch untergeordneten Bereich der Softwarearchitektur, und die Entwicklungsschicht einen daran anschließenden hierarchisch übergeordneten Bereich der Softwarearchitektur einnimmt. Mit anderen Worten ist jede Komponente der Serviceschicht, Toolkit-Schicht, Präsentationsschicht oder Web-Serviceschicht entsprechend ihrer hierarchischen Einordnung innerhalb der Softwarearchitektur auch entweder der Stabilitätsschicht oder der Entwicklungsschicht zugeordnet.

Das definierende Charakteristikum der Stabilitätsschicht besteht darin, dass die dieser Schicht zugehörenden Komponenten streng rückwärtskompatibel versioniert sind. Hierunter wird verstanden, dass - von einer Behebung eventueller Programmierfehler abgesehen - Änderungen an einer dieser Komponenten nur in dem Rahmen vorgenommen werden können, in dem ihre bisherige Funktionalität unberührt bleibt. Mit anderen Worten können Module der Stabilitätsschicht lediglich erweitert, nicht aber geändert werden. Im Gegensatz dazu besteht das definierende Charakteristikum der Entwicklungsschicht darin, dass die darin enthaltenen Module ohne den Zwang einer Rückwärtskompatibilität versionierbar sind, d.h. weiterentwickelt werden können. Durch dieses diversifizierte Versionierungskonzept wird einerseits infolge der Stabilitätsschicht sichergestellt, dass auf dem Framework aufsetzende Applikationen langfristig stabil laufen, auch wenn im Rahmen des Frameworks unterschiedlich versionierte Module eingesetzt werden oder wenn bei der Applikationsentwicklung ein subdomainspezifisches Framework zusammen mit jüngeren oder älteren Frameworkversionen anderer Sub-Domains eingesetzt wird.

Infolge der Entwicklungsschicht wird dabei gleichzeitig eine flexible Anpassungsfähigkeit des Frameworks im applikationsnahen Bereich der Framework-Architektur erzielt.

Grundsätzlich kann die Grenze zwischen der Stabilitätsschicht und der Entwicklungsschicht unabhängig von den jeweiligen Grenzen der Web-Service-, Service-, Toolkit- oder Präsentationsschicht gewählt werden. Bevorzugt ist die Grenze zwischen der Stabilitätsschicht und der Entwicklungsschicht aber derart gewählt, dass sie mit der Grenze zwischen der Serviceschicht und der Toolkit-Schicht zusammenfällt. In dieser Ausführung gehören somit die Servicekomponenten sowie - falls vorhanden - Komponenten der Web-Serviceschicht der Stabilitätsschicht an, während Toolkits und - falls vorhanden - Komponenten der Präsentationsschicht der Entwicklungsschicht angehören.

Eine besonders flexible Einsatzfähigkeit des vorstehend beschriebenen Systems wird insbesondere dadurch erreicht, dass die Module des oder jeden Frameworks derart ausgebildet sind, dass sie schichtweise gekapselt und plattformunabhängig in einer generischen Laufzeitumgebung ausführbar sind.

In zweckmäßiger Ausführung ist zu mindestens einer, bevorzugt aber zu jeder der oben aufgeführten Sub-Domains jeweils ein eigenes Framework vorgesehen. Das erfindungsgemäße System umfasst somit bevorzugt in beliebiger Auswahl oder Kombination
- ein Datenmanagement(DM)-Framework,
- ein Transfer(TF)-Framework,
- ein Aufgabenlisten(WL)-Framework,
- ein Bildbearbeitungs(IP)-Framework,
- ein Volumenbearbeitungs(VOL)-Framework,
- ein Berichterstellungs(REP)-Framework und/oder
- ein Bilderfassungs(EXAM)-Framework.

Das System umfasst ferner ein Infrastruktur-Framework, das allgemeine, im Weiteren noch näher beschriebene Funktionalitäten zur Verfügung stellt.

Das bzw. jedes Framework kann dabei eigenständig zur Anwendung kommen oder gegebenenfalls beliebig mit Frameworks anderer Sub-Domains kombiniert werden. Die beliebige Kombinierbarkeit der Frameworks, insbesondere die Einbeziehung des Bilderfassungs-Frameworks in den Framework-Pool eröffnet die Möglichkeit, neben Systemen zur Befundung auch Systeme zur Untersuchung, d.h. bilderzeugende Modalitäten, oder integrierte Untersuchungs- und Befundungssysteme zusammenzustellen. Darüber hinaus lassen sich solche Systeme auch direkt miteinander koppeln. So ist beispielsweise eine Anbindungsmöglichkeit einer Modalität an einen Bildbearbeitungs-Applikationsserver gegeben. Diese Anwendungsoptionen gehen über den Funktionsumfang herkömmlicher medizinischer Netzwerke, insbesondere über reine DICOM-Konnektivität, signifikant hinaus.

Die einzelnen Frameworks können grundsätzlich völlig unabhängig voneinander aufgebaut sein. Aus Gründen einer rationellen Erstellung und Weiterentwicklung sind im Rahmen der verschiedenen Frameworks aber für gleiche oder ähnliche Funktionalitäten bevorzugt identische Komponenten vorgesehen. Kommen mehrere derart überlappende Frameworks in Kombination zum Einsatz, so werden diese mehrfach vorhandenen Komponenten zweckmäßigerweise von den entsprechenden Frameworks gemeinsam benutzt. Im Falle von Komponenten der Service- und Web-Serviceschicht wird bevorzugt sogar ein- und dieselbe Instanz von den betreffenden Frameworks gemeinsam benutzt.

In bevorzugter Ausführung besteht das oder jedes Framework aus einem im Offline-Betrieb selbstständig lauffähigen Kern von Servicekomponenten sowie einer zugeordneten Web-Servicekomponente, die im Online-Betrieb entsprechende Dienste entfernter Netzwerkknoten zur Verfügung stellt. So sind dem Datenmanagement-Framework innerhalb der Web-Serviceschicht Remote-Datenmanagement-Dienste zugeordnet, dem Transfer-Framework sind entsprechend Remote-Transfer-Dienste zugeordnet, etc.

Für die eingangs genannten Sub-Domains haben sich die nachfolgend beschriebenen Komponentenanordnungen im Sinne einer möglichst einfachen und effektiven Softwarearchitektur, einer besonders hohen Wiederverwertbarkeit einzelner Komponenten im Rahmen verschiedener Frameworks und einer guten und flexiblen Kombinierbarkeit der verschiedenen Frameworks als besonders vorteilhaft herausgestellt. Im Sinne der besseren Übersichtlichkeit wird für Komponenten, die von mehreren Frameworks gemeinsam benutzt werden, der Funktionsumfang, die hierarchische Anordnung gegenüber bereits eingeführten Komponenten und die Abhängigkeit von untergeordneten Komponenten nur im Zusammenhang der erstmaligen Nennung näher erläutert. Sofern nicht im Einzelfall explizit anders ausgeführt, weisen diese Komponenten ihre spezifischen Eigenschaften in gleicher Weise im Rahmen aller betreffenden Frameworks auf.

### Datenmanagement(DM)-Framework:

Das DM-Framework umfasst als Servicekomponenten ein Dateizugangsmodul sowie ein Datenmanagement(DM)-Dienstmodul.

Das Dateizugangsmodul enthält fundamentale, d.h. insbesondere hardwarenahe Funktionen zum Lesen, Schreiben und Löschen von Daten auf ein bzw. von einem Dateisystem sowie zum Erzeugen von Dateien. Das Dateizugangsmodul ist dabei bevorzugt dazu ausgebildet, eine Vielzahl von unterschiedlichen Speichermedien, insbesondere eine lokale Festplatte, externe Datenspeicher, USB-Stick, etc. anzusprechen.

Das DM-Dienstmodul enthält Funktionen zur Datenauswahl sowie zur Dateizugangsregelung. Der Begriff Datenauswahl umfasst dabei sowohl die Auswahl einzelner Dateninhalte aus dem Verbund einer Datei als auch Funktionen zur Auswahl von Dateien nach vorgegebenen Suchkriterien.

Der Begriff Dateizugangsregelung umfasst insbesondere die Vergabe und Kontrolle von benutzerspezifischen Nutzungsrechten auf Dateien oder einzelne Dateiinhalte im Sinne der Datensicherheit als auch die Vergabe und Kontrolle von benutzungsspezifischen Nutzungsrechten für Dateien oder Dateiinhalte (data locking).

Innerhalb der Serviceschicht ist dabei das DM-Dienstmodul dem Dateizugangsmodul übergeordnet, wobei von dem DM-Dienstmodul aus auf das Dateizugangsmodul zugreifbar ist.

Als Toolkit umfasst das DM-Framework ein Datenmanagement(DM)-Grundmodul, das als Bestandteil einer API Funktionen zur Verfügung stellt, die einer auf dem Framework aufsetzenden Applikation einen Zugriff auf die Funktionen des DM-Dienstmoduls ermöglichen.

In einer vorteilhaften Fortentwicklung der Erfindung ist in der Serviceschicht des DM-Frameworks weiterhin ein Informations-Modell-Abbildungsmodul vorgesehen. Diese Servicekomponente enthält Funktionen, die es ermöglichen, logischen Datenobjekten eine eindeutige Referenz, und diesen Referenzen einen eindeutigen Satz von Dateien zuzuordnen, sowie derartige Referenzen zu analysieren und zu gruppieren. Als Referenz werden allgemein Daten bezeichnet, anhand derer ein Datenobjekt nach Maßgabe eines zugrunde liegenden Modells bzw. Datenstandards eindeutig identifizierbar bzw. adressierbar sowie charakterisierbar ist. Eine solche Referenz kann insbesondere in einem nach Maßgabe des Datenstandards ausgewählten Dateinamen, einer Kennnummer, od. dgl. bestehen. In zweckmäßiger Ausführung vergibt das Informations-Modell-Abbildungsmodul die Referenz auf Basis des DICOM-Standards in Form eines sogenannten UID (unique identifier).

Das Informations-Modell-Abbildungsmodul ist hierarchisch dem Dateizugangsmodul nebengeordnet. Innerhalb des Hierarchiepfads ist auf das Informations-Modell-Abbildungsmodul von dem DM-Dienstmodul aus zugreifbar.

In einer weiteren vorteilhaften Fortentwicklung der Erfindung ist in der Serviceschicht des DM-Frameworks zusätzlich ein Arbeitsbereichsmodul vorgesehen. Dieses enthält Funktionen, die die plattformunabhängige lokale Zwischenspeicherung (caching) von Daten ermöglichen.

Das Arbeitsbereichsmodul ist dem Dateizugangsmodul und - soweit vorhanden - dem Informations-Modell-Abbildungsmodul übergeordnet und kann auf dieses Modul bzw. diese Module zugreifen. Der Zugriff auf das Arbeitsbereichsmodul erfolgt seinerseits direkt ausgehend von dem DM-Grundmodul. Das Arbeitsbereichsmodul ist dem DM-Dienstmodul zweckmäßigerweise untergeordnet. Eine Zugriffsmöglichkeit ausgehenden von dem DM-Dienstmodul auf das Arbeitsbereichsmodul ist bevorzugt dennoch nicht vorgesehen, um das DM-Dienstmodul und das Arbeitsbereichsmodul voneinander unabhängig zu halten.

Zweckmäßigerweise ist dem DM-Dienstmodul und dem DM-Grundmodul eine Servicekomponente zwischengeordnet, die nachfolgend als Standard-Datenmanagement(DM)-Zugriffsmodul bezeichnet ist. Dieses Modul enthält Funktionen, die einen vereinfachten, insbesondere kombinierten Zugriff auf Funktionen des DM-Dienstmoduls und des Dateizugangsmoduls sowie - falls vorhanden - des Informations-Modell-Abbildungsmoduls ermöglichen. Beispielsweise werden häufig verwendete Operationen, die einen kombinierten Aufruf von Funktionen der vorstehend genannten untergeordneten Servicekomponenten erfordern, in dem Standard-DM-Zugriffsmodul implementiert. Auf das Standard-DM-Zugriffsmodul ist seinerseits von dem DM-Grundmodul aus zugreifbar.

Als zugeordnete Komponente der Web-Serviceschicht umfasst das DM-Framework vorzugsweise Remote-DM-Dienste. Diese Dienste ermöglichen im Online-Betrieb einen Zugriff auf Daten, die auf einem entfernten Netzwerkknoten gespeichert sind. Die Remote-DM-Dienste sind dem Dateizugangsmodul untergeordnet und von diesem ausgehend zugänglich.

Das DM-Dienstmodul umfasst in zweckmäßigen Ausbildungen der Erfindung eines oder mehrere der nachfolgend beschriebenen Sub-Systeme von Funktionen, die jeweils einem unterschiedlichen Aufgabenbereich zugeordnet sind.

So ist vorzugsweise ein erstes Sub-System (Indexing-Sub-System) von Funktionen vorgesehen, die eine Indizierung von Dateien nach Maßgabe von Meta-Daten ermöglichen. Hierbei werden insbesondere die lokal in einem Cache hinterlegten Dateien indiziert.

Als "Meta-Daten" werden Daten bezeichnet, die den hinterlegten Dateien zugeordnet sind und die diese Dateien, insbesondere deren Inhalt, näher beschreiben. Die in der medizinischen Bildgebung verarbeiteten Dateien, insbesondere digitale Bilddateien, sind regelmäßig mit einem umfangreichen Satz an Meta-Daten versehen, die z.B. Information über die bildaufnehmende Modalität, die medizinischen und technischen Aspekte einer Untersuchung, den untersuchten Patienten, etc. enthalten. Die Meta-Daten medizinischer Bildgebungs-Dateien sind insbesondere in Konformität mit dem DICOM-Standard erzeugt.

Als "Indizierung" wird in diesem Sinne jedwede Art von Indexerstellung bzw. Katalogisierung der Dateien anhand des Inhalts ihrer Meta-Daten verstanden. Diese Indizierung kann nach Maßgabe beliebig vorgegebener oder vorgebbarer Schemata erfolgen. Beispielsweise können die lokal gespeicherten Bilddaten eines bestimmten Untersuchungstyps - z.B. CT-Aufnahme - nach Maßgabe des Patientenalters indiziert werden.

Zusätzlich oder alternativ umfasst das DM-Dienstmodul ein zweites Sub-System von Funktionen (Query-Sub-System), das eine Suche unter den hinterlegten Dateien ermöglicht. Diese Suche erfolgt anhand eines Vergleichs der den hinterlegten Dateien zugeordneten Meta-Daten mit einem vorgegebenen oder vorgebbaren Suchschema. Beispielsweise kann auf diese Weise nach medizinischen Bilddaten eines bestimmten Patienten nach Maßgabe des Patientennamen durchsucht werden. Diese Suchfunktionen können als eigenständige Funktionalität ausgebildet sein. In diesem Fall werden die Meta-Daten der hinterlegten Dateien direkt durchsucht. Für eine vereinfachte und somit auch schnellere Suche sind die Funktionen des Query Sub-Systems alternativ dazu ausgebildet, auf der vorstehend beschriebenen Indizierung der Dateien aufzubauen. Beide Varianten - Direktsuche und indexgestützte Suche - können auch miteinander kombiniert sein.

Wiederum zusätzlich oder alternativ umfasst das DM-Dienstmodul zwei weitere Sub-Systeme (Synchronisation-Sub-System, und Locking-Sub-System) von Funktionen, die der Synchronisierung von Dateien und/oder Vermeidung eines Mehrfachzugangs auf dieselbe Datei dienen. Unter "(Datei-)Synchronisierung" wird im Hinblick auf eine Datei, die in mehreren Kopien an unterschiedlichen Speicherorten hinterlegt ist, ein Abgleichautomatismus verstanden, durch den verhindert wird, dass sich der Dateiinhalt der Kopien infolge Änderungen an jeweils einer Kopie sukzessiv auseinanderentwickelt. Insbesondere werden durch die Synchronisierung Änderungen, die lokal und offline an einer Datei vorgenommen wurden, bei der Wiederaufnahme des Online-Betriebs auf die an anderer Stelle des medizinischen Netzwerkes abgelegten Kopien dieser Datei automatisch übertragen.

Thematisch eng verknüpft mit der Dateisynchronisierung ist die Vermeidung eines gleichzeitigen Mehrfachzugangs auf die selbe Datei im Online-Betrieb. Derartige Funktionen werden insbesondere auch als "Data-Locking" bezeichnet. Im Zuge des Data-Locking werden insbesondere Schreib- und Löschrechte anderer Benutzer an einer Datei gesperrt, sobald und solange ein Benutzer diese Datei bearbeitet. Um bei Dateien, die in verschiedenen Kopien an unterschiedlichen Speicherorten hinterlegt sind, eine parallele Bearbeitung der verschiedenen Kopien effektiv zu verhindern, ist bevorzugt vorgesehen, dass globale, d.h. netzwerküberspannende Reservierungen für eine Datei vorgenommen werden können. Um eine effektive und konfliktfreie Bearbeitung eines komplexen Datenobjekts, das eine Vielzahl von Einzeldateien in einer vorgegebenen hierarchischen Ordnung enthält, sicherzustellen, ist zweckmäßigerweise vorgesehen, dass das Locking-Sub-Set auf bestimmten Hierarchiestufen des Datenobjekts Reservierungen vornehmen kann. Ein solches komplexes Datenobjekt ist insbesondere eine elektronische Patientenakte, die in der z.B. durch DICOM vorgegebenen hierarchischen Struktur Patientengrunddaten, Behandlungsabläufe, Untersuchungen, Bilder, Befundungsberichte, etc. enthält, wobei durch das Locking-Sub-Set z.B. wahlweise die gesamte Patientenakte oder auch nur eine bestimmte Untersuchung für anderweitigen Zugriff blockiert werden kann.

Wiederum zusätzlich oder alternativ umfasst das DM-Dienstmodul ein weiteres Sub-System (Security-Sub-System) von Funktionen, das für die hinterlegten Dateien oder spezielle Dateiinhalte dieser Dateien, insbesondere einen Datei-Header, benutzerspezifische Benutzungsrechte vergibt und kontrolliert. Hierdurch wird ein unbefugter Zugang bzw. eine unbefugte Manipulation der hinterlegten Dateien bzw. Dateiinhalte vermieden.

Wiederum zusätzlich oder alternativ umfasst das DM-Dienstmodul schließlich ein weiteres Sub-System (Work-Flag-Sub-System) von Funktionen, das der Verwaltung so genannter Work-Flags, die hinterlegten Dateien zugeordnet sind, dient. Workflags definieren Regeln, die für die zugeordneten Dateien automatisierte Datenverarbeitungsprozesse, z.B. Auto-Archivierung, Auto-Transfer oder Auto-Druck, steuern.

### Transfer(TF)-Framework:

Das TF-Framework umfasst in seiner Grundstruktur als ServiceKomponenten neben dem bereits im Zusammenhang mit dem DM-Framework beschriebenen Dateizugangsmodul ein Pipeline-Modul und ein Transfer(TF)-Dienstmodul.

Das Pipeline-Modul bildet einen Kanal bzw. Rahmen für die Bearbeitung von Bilddaten sowie ferner diesen zugeordneten Meta-Daten. Es spezifiziert dabei in zweckmäßigerweise Ausgestaltung eine Anzahl von (physischen oder logischen) Quellgeräten und eine Anzahl von (physischen oder logischen) Zielgeräten für Bilddaten sowie darüber hinaus eine Anzahl von Bildbearbeitungs-Filtern, die den Quellgeräten und den Zielgeräten einzeln oder in beliebiger Kombination zwischengeschaltet werden können und die die durch die Pipeline geleiteten Bilddaten modifizieren. Als Filter können der Pipeline beliebige Bild- und Volumenbearbeitungsfilter, z.B. für Helligkeits- und Kontrastoperationen, Farbänderungen, Glättung, Scharfzeichnung, Drehung, Streckung, Stauchung, etc. zugeordnet werden. Ferner können der Pipeline auch Filter für Meta-Daten zugeordnet werden.

Das TF-Dienstmodul enthält Funktionen zur Übertragung von Daten zwischen zwei Dateisystemen unter Zugriff auf das Dateizugangsmodul und/oder das Pipeline-Modul.

Als Komponente der Toolkit-Schicht umfasst das TF-Framework ein Transfer(TF)-Grundmodul, das als Bestandteil einer API Funktionen zur Verfügung stellt, die einer auf dem Framework aufsetzenden Applikation einen Zugriff auf die Funktionen des TF-Dienstmoduls ermöglichen. Das TF-Grundmodul ist dabei insbesondere einer Master-Seite der Programmierumgebung der Toolkit-Schicht zuordenbar.

Im Zuge einer vorteilhaften Erweiterung umfasst das TF-Framework optional als weitere Komponente der Serviceschicht das Informations-Modell-Abbildungsmodul, das Arbeitsbereichs-modul und/oder das Standard-DM-Zugriffsmodul.

Im Rahmen des TF-Frameworks ist dabei von dem Pipeline-Modul aus auf das Arbeitsbereichsmodul aus zugreifbar. Auf die Funktionalität des Standard-DM-Zugriffsmoduls kann im Rahmen des TF-Frameworks von dem TF-Grundmodul aus zugegriffen werden. Das Standard-DM-Zugriffsmodul enthält für den Einsatz im Rahmen des TF-Frameworks Funktionen zum vereinfachten, insbesondere kombinierten Zugriff auf Funktionen des TF-Dienstmoduls, des Dateizugriffsmoduls und/oder - falls vorgesehen - des Informations-Modell-Abbildungsmoduls.

Im Rahmen des TF-Frameworks (sowie auch im Rahmen der nachfolgend beschriebenen Frameworks) ist optional die Möglichkeit vorgesehen, als weitere Komponenten der Toolkit-Schicht ein oder mehrere "Baukasten-Module" (im Rahmen des TF-Frameworks als Transfer(TF)-Baukastenmodule bezeichnet) zu erzeugen, die jeweils einer Inhaltsseite der Programmierumgebung zuordenbar sind. Dies ermöglicht einem Applikationsentwickler, den Funktionsumfang des jeweiligen Frameworks bei Bedarf zu erweitern, wobei er hierzu u.a. baukastenartig auf Funktionalitäten der Serviceschicht zugreifen kann. Die Baukastenmodule gehören somit nicht zur Grundausstattung des jeweiligen Frameworks und haben auch keinen vordefinierten Funktionsinhalt, sondern können nach Bedarf erstellt und mit der kundenspezifisch gewünschten Funktionalität ausgestattet werden.

Als zugeordnete Komponente der Web-Serviceschicht umfasst das TF-Framework Remote-TF-Dienste, die einen Datentransfer mit entfernten Netzwerkknoten ermöglichen. Die Remote-TF-Dienste sind dem Dateizugangsmodul untergeordnet und von diesem ausgehend zugänglich.

### Interaktion zwischen DM-Framework und TF-Framework

Wird das Standard-DM-Zugriffsmodul isoliert im Rahmen entweder des DM-Frameworks oder des TF-Frameworks eingesetzt, kann jeweils nur ein frameworkspezifisch eingeschränkter Funktionsrahmen dieses Moduls genutzt werden. Darüber hinaus umfasst das Standard-DM-Zugriffsmodul aber vorzugsweise auch Funktionen zum kombinierten Zugriff auf spezifische Funktionen des DM-Frameworks (insbesondere Funktionen des DM-Dienstmoduls) und spezifische Funktionen des TF-Frameworks (insbesondere Funktionen des TF-Dienstmoduls). Um diese frameworkübergreifenden Zugriffsmöglichkeiten zu nutzen, werden das DM-Framework und das TF-Framework bevorzugt als funktionelle Einheit in Kombination eingesetzt, wobei in diesem Fall sowohl das DM-Grundmodul als auch das TF-Grundmodul auf das Standard-DM-Zugriffsmodul zugreifen.

Das Standard-DM-Zugriffsmodul bildet ferner eine vielseitig einsetzbare und praktische Schnittstelle, über auch die Toolkits weiterer Frameworks einfach auf gängige Funktionalitäten des DM- und TF-Frameworks zugreifen können.

### Aufgabenlisten(WL)-Framework

Das WL-Framework umfasst in seinem Grundgerüst als Komponenten der Serviceschicht neben dem Dateizugangsmodul und dem hierauf zugreifenden Arbeitsbereichsmodul ein dem Arbeitsbereichsmodul übergeordnetes Aufgabenlisten(WL)-Dienstmodul. Das WL-Dienstmodul enthält Funktionen zur Verknüpfung von Daten mit Aufgabenlisteneinträgen. Es ist hierzu zum Zugriff auf das Arbeitsbereichsmodul ausgebildet.

Als die z.B. einer Master-Seite der Programmierumgebung zuordenbare Komponente der Toolkit-Schicht weist das WL-Framework ein Aufgabenlisten(WL)-Grundmodul, das als Bestandteil der API Funktionen enthält, die einer auf dem Framework aufsetzenden Applikation einen Zugriff auf die Funktionen des WL-Dienstmoduls ermöglichen.

Auch im Rahmen des WL-Frameworks ist optional die Möglichkeit vorgesehen, als weitere Komponenten der Toolkit-Schicht ein oder mehrere kundenspezifisch generierbare Worklist(WL)-Baukasten-Module zu erzeugen, die jeweils einer Inhaltsseite der Programmierumgebung zuordenbar sind.

Das WL-Framework verfügt bevorzugt auch über höherrangige Funktionalitäten des Datenmanagements und/oder Datentransfers. Auf diese Funktionalitäten greift das WL-Grundmodul über das Standard-DM-Zugriffsmodul zu. In dieser Variante umfasst das WL-Framework aufgrund der Abhängigkeiten des Standard-DM-Zugriffsmoduls auch das DM-Dienstmodul und/oder das TF-Dienstmodul mit dem diesem wiederum untergeordneten Pipeline-Modul.

Als Komponente der Web-Serviceschicht umfasst das WL-Framework weiterhin bevorzugt Remote-Worklist(WL)-Dienste, die Funktionen zur netzwerküberspannenden Koordination von Aufgabenlisten, z.B. im Rahmen eines sogenannten Informationssystems enthalten. Auf die Remote-WL-Dienste ist dabei direkt von dem WL-Grundmodul aus zugreifbar.

### Bildbearbeitungs(IP)-Framework

Das IP-Framework umfasst in seinem Grundgerüst als Komponenten der Serviceschicht neben dem Pipeline-Modul ein diesem untergeordnetes Segmentierungsmodul, ein dem Pipeline-Modul nebengeordnetes Graphik/Anzeige-Modul sowie ein dem Pipeline-Modul und dem Graphik/Anzeige-Modul übergeordnetes Bildbearbeitungs(IP)-Dienstmodul.

Das Segmentierungsmodul umfasst Funktionen zur Segmentierung von medizinischen Bilddaten. Das Graphik/Anzeige-Modul enthält Funktionen zur Handhabung und Anzeige von geschichteten Bildsegmentinhalten, insbesondere zur interaktiven Bildmanipulation, z.B. zur Vermessung von Bildsegmenten am Bildschirm. Sowohl von dem Pipeline-Modul als auch von dem Graphik/Anzeige-Modul aus ist im Rahmen des IP-Frameworks das auf das Segmentierungsmodul zugreifbar.

Das IP-Dienstmodul umfasst Funktionen zur Anzeige medizinischer Bilddaten. Die Funktionen des IP-Dienstmoduls können hierzu auf Funktionen des Pipeline-Moduls, des Anzeige/Graphik-Moduls und/oder des Segmentierungsmoduls zugreifen.

Als die z.B. einer Master-Seite der zugehörigen Programmierungsoberfläche zugeordnete Komponente der Toolkit-Schicht umfasst das IP-Framework ein Bildanzeige-Grundmodul, das als Bestandteil der API einer auf dem Framework aufsetzenden Applikation Funktionen zum Zugriff auf das IP-Dienstmodul zur Verfügung stellt.

Auch im Rahmen des IP-Frameworks ist optional die Möglichkeit vorgesehen, als weitere Komponenten der Toolkit-Schicht ein oder mehrere kundenspezifisch generierbare IP-Baukasten-Module zu erzeugen, die jeweils einer Inhaltsseite der Programmierumgebung zuordenbar sind.

Als Komponente der Web-Serviceschicht umfasst das IP-Framework weiterhin bevorzugt Remote-Display-Dienste, die Funktionen zur Anzeige von geschichteten Bildsegmentinhalten auf einem entfernten Knoten eines medizinischen Netzwerkes umfassen. Der Zugriff auf die Funktionen der Remote-Display-Dienste erfolgt hierbei von dem Graphik/Anzeige-Modul aus.

In einer bevorzugten Variante nutzt das IP-Framework für Funktionalitäten des Datenmanagements die grundlegenden Komponenten des DM-Frameworks, nämlich das Dateizugangsmodul und/oder das Informations-Modell-Abbildungsmodul mit. Der Zugriff auf diese Funktionalitäten erfolgt ausgehend von dem Pipeline-Modul mittelbar über das Arbeitsbereichsmodul. Auf letzteres kann das IP-Dienstmodul optional auch direkt zugreifen. Ferner verfügt das IP-Framework auch über höherrangige Funktionalitäten des Datenmanagement und/oder Datentransfers, auf die das IP-Grundmodul in zweckmäßiger Ausgestaltung über das Standard-DM-Zugriffsmodul zugreift. In diesen Varianten umfasst das IP-Framework aufgrund der Abhängigkeiten des Standard-DM-Zugriffsmoduls auch das DM-Dienstmodul und/oder das TF-Dienstmodul.

### Volumenbearbeitungs(VOL)-Framework

Das VOL-Framework umfasst in seinem Grundgerüst als Komponenten der Serviceschicht neben dem Dateizugangsmodul (sowie optional dem nebengeordneten Informations-Modell-Abbildungsmodul) und dem auf das Dateizugangsmodul zugreifenden Arbeitsbereichsmodul, dem Segmentierungsmodul und dem hierauf zugreifenden Graphik/Anzeige-Modul ein dem letzteren übergeordnetes Volumenbearbeitungs(VOL)-Dienstmodul.

Das VOL-Dienstmodul enthält Funktionen zur Bearbeitung und Anzeige von medizinischen Volumendaten unter Zugriff auf das Anzeige/Graphik-Modul und/oder das Arbeitsbereichsmodul.

Als die z.B. einer Master-Seite der Programmierumgebung zuordenbare Komponente der Toolkit-Schicht umfasst das VOL-Framework ein Bildanzeige-Grundmodul, das als Bestandteil der API einer auf dem Framework aufbauenden Applikation Funktionen zum Zugriff auf das VOL-Dienstmodul zur Verfügung stellt.

Auch im Rahmen des VOL-Frameworks ist optional die Möglichkeit vorgesehen, als weitere Komponenten der Toolkit-Schicht ein oder mehrere kundenspezifisch generierbare Volumenbearbeitungs(VOL)-Baukasten-Module zu erzeugen, die jeweils einer Inhaltsseite der Programmierumgebung zuordenbar sind.

Zumal auch die Darstellung von Volumendaten lediglich in Form zweidimensionaler Graphiken - insbesondere räumlichen Ansichten und/oder Schnittdarstellungen - möglich ist, erfordert die Darstellung der Volumendaten erkanntermaßen eine mit der Sub-Domain der Bildbearbeitung weitgehend identische Funktionalität. Im Sinne einer besonders rationalen Softwarearchitektur werden daher zweckmäßigerweise die auf die Bildanzeige gerichteten Komponenten des Systems von dem IP-Framework und dem VOL-Framework gemeinsam benutzt. So ist in bevorzugter Ausbildung neben dem Segmentierungsmodul und dem Graphik/Anzeigemodul mit dem Bildanzeige-Grundmodul für beide Frameworks auch ein identisches Toolkit vorgesehen.

Das VOL-Framework umfasst bevorzugt als weitere Komponente der Serviceschicht ein 3D-Bildsynthese-Modul. Das 3D-Bildsynthese-Modul enthält Funktionen zur Erstellung räumlicher Bilder anhand von 3D-Szenen. Diese Funktionalität wird üblicherweise auch als "volume rendering" bezeichnet.

Als Komponenten der Web-Serviceschicht umfasst das VOL-Framework optional weiter VOL-Remote-Dienste sowie weiterhin optional die Remote-Display-Dienste. Erstere umfassen Funktionen zur Bearbeitung von Volumendaten auf einem entfernten Knoten des medizinischen Netzwerkes. Auf die VOL-Remote-Dienste kann dabei von dem VOL-Dienstmodul aus zugegriffen werden.

Ferner verfügt das VOL-Framework auch über höherrangige Funktionalitäten des Datenmanagement und/oder Datentransfers, auf die das VOL-Grundmodul in zweckmäßiger Ausgestaltung über das Standard-DM-Zugriffsmodul zugreift. In diesen Varianten umfasst das VOL-Framework aufgrund der Abhängigkeiten des Standard-DM-Zugriffsmoduls auch das DM-Dienstmodul und/oder das TF-Dienstmodul.

### Berichterstellungs(REP)-Framework

Das REP-Framework umfasst als Komponenten der Serviceschicht neben dem Dateizugangsmodul und dem auf das Dateizugangsmodul zugreifenden Arbeitsbereichsmodul ein diesem übergeordnetes Berichterstellungs(REP)-Dienstmodul.

Das REP-Dienstmodul enthält Funktionen zum Erstellen und automatisierten Lesen von nach Maßgabe eines Datenstandards, insbesondere DICOM, strukturierten Berichten unter Zugriff auf das Arbeitsbereichsmodul.

In bevorzugter Ausführung greift das REP-Dienstmodul zudem auf diesem innerhalb der Serviceschicht untergeordnetes Terminologiemodul zu, das auf lokaler Ebene Textbausteine für eine vereinheitlichte Berichterstellung zur Verfügung stellt.

Dem Terminologiemodul ist optional eine nachfolgend als Zentral-Terminologie-Service bezeichnete Komponente der Web-Serviceschicht untergeordnet, die im Online-Betrieb netzwerkweit entsprechende Textbausteine zur Verfügung stellt.

Als Komponente der Toolkit-Schicht umfasst das REP-Framework ein Berichterstellungs(REP)-Grundmodul, das als Bestandteil der API Funktionen zur Verfügung stellt, die einer auf dem Framework aufbauenden Applikation den Zugriff auf das REP-Dienstmodul ermöglichen.

Auch im Rahmen des REP-Frameworks ist optional die Möglichkeit vorgesehen, als weitere Komponenten der Toolkit-Schicht ein oder mehrere kundenspezifisch generierbare Berichterstellungs(REP)-Baukasten-Module zu erzeugen, die jeweils einer Inhaltsseite der Programmierumgebung zuordenbar sind.

Ferner verfügt das REP-Framework auch über höherrangige Funktionalitäten des Datenmanagement und/oder Datentransfers, auf die das REP-Grundmodul in zweckmäßiger Ausgestaltung über das Standard-DM-Zugriffsmodul zugreift. In diesen Varianten umfasst das REP-Framework aufgrund der Abhängigkeiten des Standard-DM-Zugriffsmoduls auch das DM-Dienstmodul und/oder das TF-Dienstmodul sowie das Pipeline-Modul.

### Bilderfassungs(EXAM)-Framework

Das EXAM-Framework umfasst in seiner Grundstruktur als Komponente der Serviceschicht ein Bilderfassungs(EXAM)-Dienstmodul, das Funktionen zur Erfassung von Rohdaten unter Zugriff auf mindestens eine medizinische Bildquelle sowie zur Aufbereitung dieser Rohdaten zu medizinischen Bilddaten und zur Echtzeit-Anzeige (Life-Display) und Archivierung von Rohdaten und/oder aufbereiteten Bilddaten enthält.

Als Komponente der Toolkit-Schicht umfasst das EXAM-Framework ein Bilderfassungs(EXAM)-Grundmodul, das als Bestandteil der API Funktionen zur Verfügung stellt, die einer auf dem Framework aufbauenden Applikation den Zugriff auf die Funktionalität des EXAM-Dienstmoduls ermöglicht.

In einer vorteilhaften Erweiterung umfasst das EXAM-Framework Remote-Bilderfassungs(EXAM)-Dienste, die für den Online-Betrieb Funktionen zur Erfassung von Rohdaten unter Zugriff auf mindestens eine entfernte medizinische Bildquelle und/oder zur Aufbereitung dieser Rohdaten auf einem entfernten Knoten eines medizinischen Netzwerkes umfasst. Auf die Remote-EXAM-Dienste, die als Komponente der Web-Serviceschicht eingeordnet sind, ist dabei von dem EXAM-Dienstmodul aus zugreifbar.

Für grundlegende Funktionen des Datenmanagements und Datentransfers nutzt das EXAM-Framework bevorzugt die entsprechenden Komponenten des DM- und/oder TF-Frameworks, nämlich das Dateizugangsmodul und/oder das Informations-Modell-Abbildungsmodul, mit. Das EXAM-Dienstmodul umfasst dabei Funktionen, die direkt auf das Dateizugangsmodul bzw. das Informations-Modell-Abbildungsmodul zugreifen. Darüber hinaus umfasst das EXAM-Dienstmodul optional auch Funktionen, die auf höherer Ebene, nämlich über das Arbeitsbereichs-Modul, auf die Funktionalität des DM- bzw. TF-Frameworks zugreifen. Optional ist weiterhin eine Zugriffsmöglichkeit des EXAM-Grundmoduls auf das (wiederum von dem DM- bzw. TF-Dienstmodul anhängige) Standard-DM-Zugriffsmodul vorgesehen.

Eine wesentliche Aufgabe des EXAM-Frameworks besteht darin, die von den Bildquellen erzeugten Rohdaten (bei denen es sich in der Regel bereits ihrer Natur nach um Bilddaten handelt) zum Zweck der Archivierung oder zum Zweck der Echtzeit- bzw. Life-Anzeige mit Mitteln der Bildbearbeitung aufzubereiten. Dies erfordert erkanntermaßen eine ähnliche Funktionalität, wie sie im Rahmen des IP-Frameworks erforderlich ist. Das EXAM-Framework nutzt daher in einer bevorzugten Variante für die genannten Zwecke die entsprechenden Servicekomponenten des IP-Frameworks, nämlich das Segmentierungsmodul, das Pipeline-Modul, das Graphik/Anzeige-Modul (gegebenenfalls mit den diesen optional untergeordneten Remote-Display-Diensten), und das IP-Dienstmodul, mit. Das IP-Dienstmodul ist dabei dem EXAM-Dienstmodul neben- oder untergeordnet. Der Zugriff auf die Funktionen des IP-Dienstmoduls erfolgt bevorzugt direkt von dem EXAM-Grundmodul aus. Das EXAM-Dienstmodul enthält darüber hinaus Funktionen, die direkt auf das Graphik/Anzeige-Modul und/oder das Segmentierungsmodul zugreifen.

Zur Echtzeit-Anzeige räumlicher Graphiken, wie sie beispielsweise im Zusammenhang mit einem Computer- oder MR-Tomographen als Bildquelle zur Anzeige der Untersuchungsdaten erforderlich ist, greift das EXAM-Dienstmodul weiterhin zweckmäßigerweise auf das (die entsprechende Funktionalität zur Verfügung stellende) 3D-Bildsynthese-Modul des VOL-Frameworks zu.

Das EXAM-Dienstmodul umfasst bevorzugt ein Bildquellen-Kontroll-Sub-System von Funktionen, die die Kommunikation mit einer Anzahl definierter Bildquellen ermöglicht. Der Begriff Kommunikation bezeichnet hierbei insbesondere das Senden und Empfangen von Steuerbefehlen bzw. entsprechenden Antworten sowie weiteren damit zusammenhängenden Daten. Das Bildquellen-Kontroll-Sub-System ermöglicht z.B., Aufnahmeparameter an eine bestimmte Bildquelle zu senden und in Antwort hierauf die entsprechend aufgenommenen Rohdaten inklusive der zugehörigen Meta-Daten zu empfangen.

Das EXAM-Dienstmodul umfasst zusätzlich oder alternativ ein Rohdaten-Anpassungs-Subsystem von Funktionen, die eine Anpassung der bildquellenspezifischen Rohdatenformate, insbesondere hinsichtlich der jeweils zugeordneten Meta-Daten, an einen einheitlichen Datenstandard, insbesondere DICOM, vornehmen.

Beiden Sub-Systemen liegt die Erkenntnis zugrunde, dass die in einem medizinischen Netzwerk enthaltenen Bildquellen üblicherweise unterschiedliche Gerätetypen mehrerer Hersteller und unterschiedlichen Technologiestandards umfassen, mit anderen Worten äußerst uneinheitlich sind. Diese Uneinheitlichkeit äußert sich erkanntermaßen in der Regel auch darin, dass die Kommunikation mit jeder Bildquelle auf eine individuelle Weise erfolgen muss, und dass auch die von den einzelnen Bildquellen ausgegebenen Rohdaten, insbesondere hinsichtlich der jeweiligen Header-Formate individuelle Züge tragen.

Die oben beschriebenen Sub-Systeme fungieren als Übersetzer zwischen den einheitlichen Steuer- und Anzeigefunktionen des EXAM-Frameworks und den individuellen Anforderungen der einzelnen Bildquellen. Um neue Bildquellen auf einfache Weise in das System integrieren zu können, sind die Funktionen dieser Sub-Systeme bevorzugt als bildquellenspezifische Plugins konzipiert, so dass beide Sub-Systeme ohne großen Installationsaufwand, insbesondere ohne Neukompilierung, angepasst werden können. Diese Konzeption ermöglicht insbesondere auch dem Bildquellenhersteller, die entsprechenden Plugins ähnlich einem Hardwaretreiber zusammen mit der Bildquelle mitzuliefern.

Das Infrastruktur-Framework umfasst als Komponenten der Serviceschicht ein Benutzerverwaltungs- bzw. Sicherheitsmodul, das Funktionen zur Authentisierung und Authorisierung von Benutzern sowie zum Audit-Trail-Handling ebenso enthält wie auf das Benutzer- und Rollenmanagement gerichtete Funktionen, ein Konfigurationsmanagement-Modul für jegliche Zwecke der Konfiguration einer Applikation, ein Lizenzmanagement-Modul, das Funktionen zur Unterstützung der Lizenzierung von Applikationen oder deren Komponenten-Funktionalität enthält, ein Error-Managementmodul, das Funktionen zur Unterstützung einer einheitlichen Fehlerbearbeitung und Fehleranzeige enthält, ein Trace-Management-Modul, das Funktionen zur Unterstützung eines einheitlichen Trace-Handlings enthält, d.h. anzeigt, welche Funktion eines Modules durchlaufen worden ist, ein Benutzungs-Managementmodul, das Funktionen zur Unterstützung eines einheitlichen Benutzungs-Handlings enthält, d.h. angibt, in welcher Form das System oder eine Applikation verwendet worden ist.

Als Komponenten der Web-Serviceschicht umfasst das Infrastruktur-Framework Infrastruktur-Remote-Dienste, die die entsprechenden Funktionalitäten des Benutzer- und Sicherheitsmanagement, Konfigurationsmanagement, Lizenzmanagement, Error-Management, Trace-Handling, und Benutzungsmanagement im Online-Betrieb netzwerkweit zur Verfügung stellen.

Nachfolgend werden Ausführungsbeispiele der Erfindung anhand einer Zeichnung näher erläutert. Darin zeigen
- FIG 1: in schematischer Darstellung allgemein eine Schichtstruktur eines System zur Erzeugung und zum Betrieb einer Softwareapplikation für die Informationsverarbeitung in der Domain der medizinischen Bildgebung mit mindestens einem subdomainspezifischen Framework,
- FIG 2: in einem schematischen Blockschaltbild auf Basis des Schemas gemäß FIG 1 eine erste Ausführung des Systems mit einem Datenmanagement-Framework,
- FIG 3: in Darstellung gemäß FIG 2 eine alternative Ausführung des Systems mit einem Transfer-Framework,
- FIG 4: in Darstellung gemäß FIG 2 eine weitere Ausführung des Systems, die das Datenmanagement-Framework gemäß FIG 2 und das Transfer-Framework gemäß FIG 3 in Kombination enthält,
- FIG 5: in Darstellung gemäß FIG 2 eine weitere Ausführung des Systems mit einem Aufgabenlisten-Framework,
- FIG 6: in Darstellung gemäß FIG 2 eine weitere Ausführung des Systems mit einem Bildbearbeitungs-Framework,
- FIG 7: in Darstellung gemäß FIG 2 eine weitere Ausführung des Systems mit einem Volumenbearbeitungs-Framework,
- FIG 8: in Darstellung gemäß FIG 2 eine weitere Ausführung des Systems, die das Bildbearbeitungs-Framework gemäß FIG 6 und das Volumenbearbeitungs-Framework gemäß FIG 7 in Kombination enthält,
- FIG 9: in Darstellung gemäß FIG 2 eine weitere Ausführung des Systems mit einem Berichterstellungs-Framework,
- FIG 10: in Darstellung gemäß FIG 2 eine weitere Ausführung des Systems mit einem Bilderfassungs-Framework, und
- FIG 11: in Darstellung gemäß FIG 2 eine weitere Ausführung des Systems, die das Datenmanagement-Framework gemäß FIG 2, das Transfer-Management gemäß FIG 3, das Aufgabenlisten-Framework gemäß FIG 5, das Bildbearbeitungs-Framework gemäß FIG 6 und das Volumenbearbeitungs-Framework gemäß FIG 7 und das Berichterstellungs-Framework gemäß FIG 9 in Kombination enthält.

Einander entsprechende Strukturen sind in allen Figuren stets mit gleichen Bezugszeichen versehen.

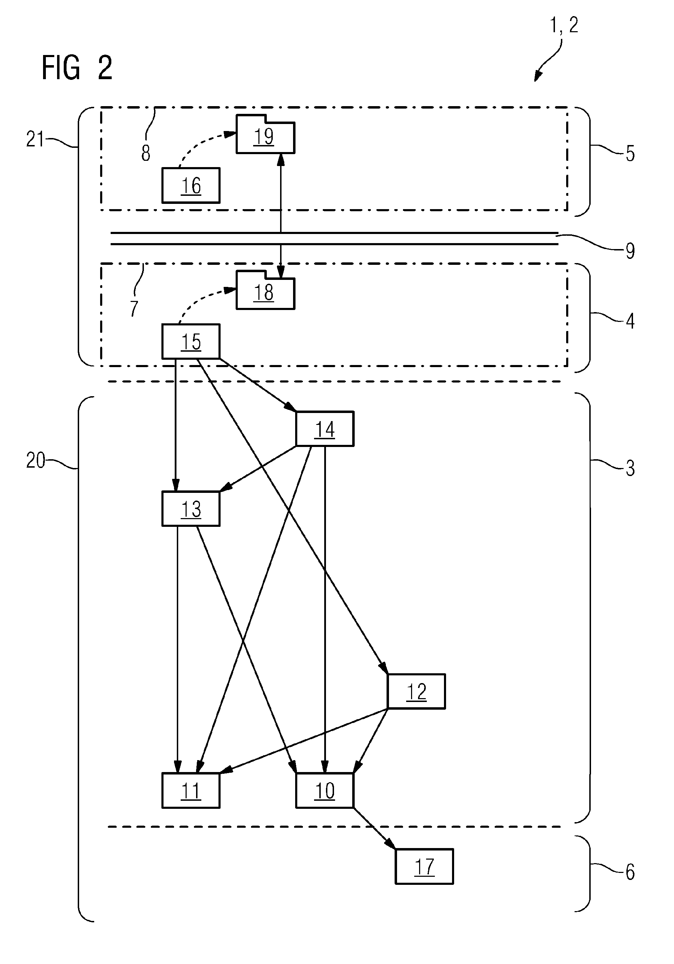

Die FIG 2 bis 11 zeigen in verschiedenen Ausführungen ein System 1 zur Erzeugung sowie zum Betrieb einer Softwareapplikation für die Domain der medizinischen Bildgebung, das - je nach Ausführung - ein sub-domain-spezifisches Framework oder mehrere solcher Frameworks in Kombination enthält. Alle nachfolgend beschriebenen Frameworks sind nach den gleichen grundlegenden Strukturprinzipien, insbesondere nach einem gemeinsamen Schichtaufbau und einem streng-hierarchischen Abhängigkeitsprinzip, konzipiert. Die nachfolgend beschriebenen Frameworks überlappen - mehr oder weniger ausgeprägt - hinsichtlich einiger Komponenten. Diese für alle betreffenden Frameworks stets gleich benannten Komponenten sind - sofern nicht explizit anders ausgeführt - im Rahmen eines jeden betreffenden Frameworks hinsichtlich ihres Funktionsumfangs, ihrer hierarchischen Anordnung und ihrer Abhängigkeit von anderen gemeinsamen Komponenten der Frameworks identisch.

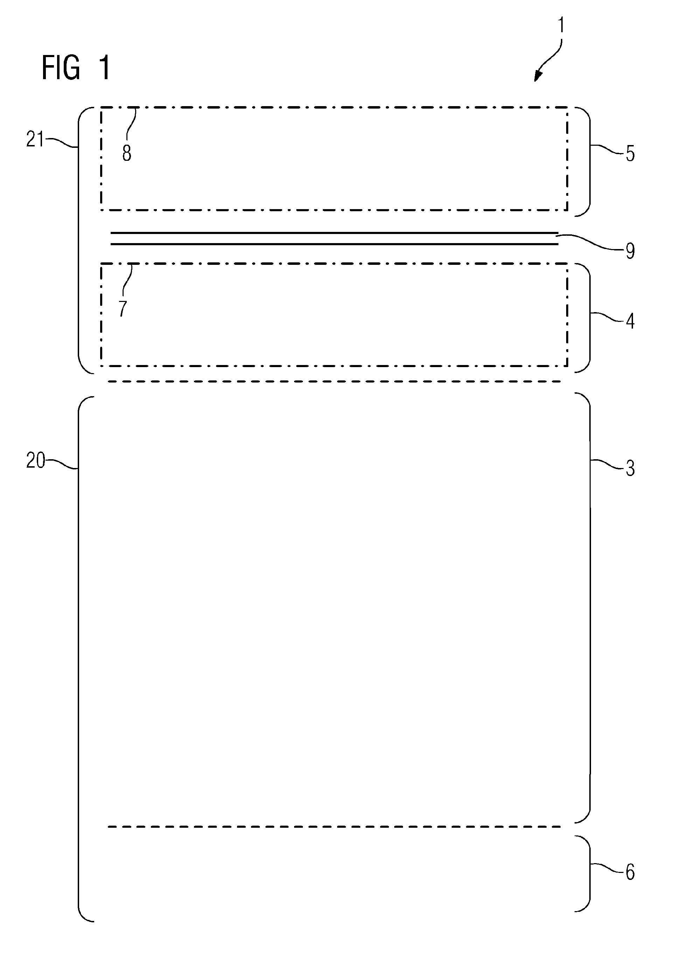

Die gemeinsamen Strukturprinzipien der Frameworks werden nachfolgend anhand eines in FIG 1 dargestellten Schemas näher erläutert, das die Schichtanordnung, auf der alle Frameworks des Systems 1 beruhen, zunächst abstrakt zeigt.

Das (bzw. jedes) Framework umfasst mehrere Softwareschichten. Der Kernbereich des Frameworks wird dabei gebildet durch eine Serviceschicht 3 sowie eine dieser übergeordnete Toolkit-Schicht 4. Das Framework umfasst des Weiteren eine Präsentationsschicht 5 sowie eine Web-Serviceschicht 6, die der Serviceschicht 3 untergeordnet ist.

Die Präsentationsschicht 5 bildet für eine auf dem System 1 aufbauende Softwareapplikation die Schnittstelle zwischen den Prozessen der Softwareapplikation und einem Benutzer. Sie dient dabei insbesondere zum Aufbau einer graphischen Benutzeroberfläche (auch als GUI, graphic user interface, bezeichnet) .

Die Serviceschicht 3 enthält - allgemein gesprochen - "Dienste", die der Softwareapplikation durch das Framework zur Verfügung gestellt werden. Die Toolkit-Schicht 4 ermöglicht der Softwareapplikation den Zugriff auf diese Dienste. Die Toolkit-Schicht 4 vermittelt dabei insbesondere als mit der Präsentationsschicht 5 korrespondierendes Backend die informationstechnische Verknüpfung der Präsentationsschicht 5 mit der Serviceschicht 3.

Die Toolkit-Schicht 4 bildet ein API, und damit die für einen Applikationsentwickler sichtbare Oberfläche des Frameworks, auf der die eigentliche Softwareapplikation unter Nutzung der für den Applikationsentwickler in der Regel nicht direkt zugänglichen Funktionen der Serviceschicht 3 aufgebaut werden kann. Zur Ermöglichung einer schnellen Applikationsentwicklung stellt die Toolkit-Schicht 4 eine visuelle Programmierumgebung 7 zur Verfügung, auf der Komponenten der Toolkit-Schicht 4 graphisch instanziert und über Methoden und Ereignisse miteinander verknüpft bzw. "verdrahtet" werden können. Die Programmierumgebung 7 ist dabei in Form von Seiten gegliedert. Sie stellt dabei für das oder jedes Framework eine Master-Seite zur Verfügung, auf der durch entsprechende Verknüpfung von Komponenten der Toolkit-Schicht 4 ein Rahmen für die framework-spezifischen Eigenschaften einer Applikation festgelegt werden kann. Diese Master-Seite kann optional im Rahmen mehrerer der nachfolgend beschriebenen Frameworks als Vorlage zur Erstellung sogenannter Inhaltsseiten herangezogen werden, die zusätzlich zu den Eigenschaften der Master-Seite einen jeweils verschiedenen Inhalt aufweisen.

Korrespondierend hierzu stellt auch die Präsentationsschicht 5 eine visuelle Programmierumgebung 8 zu Verfügung, auf der die graphische Benutzeroberfläche aufbaubar ist, und die analog zu der Programmierumgebung 7 in Seiten gegliedert ist.

Der Datenaustausch zwischen der Toolkit-Schicht 4 und der Präsentationsschicht 5 erfolgt über eine Verschaltungsschnittstelle 9. Die Verschaltungsschnittstelle 9 ist flexibel konfigurierbar derart, dass die Verbindung zwischen Toolkit-Schicht 4 und Präsentationsschicht 5 automatisch an verschiedene Deployments dieser Schichten 4,5 auf einen oder mehrere Rechner anpassbar ist. Die Verschaltungsschnittstelle 9 ermöglicht also, Frontend und Backend einer auf das System 1 aufsetzenden Softwareapplikation wahlweise auf demselben Rechner (als sogenannte Desk-top-Applikation) oder verteilt auf mehreren Rechner (Client-Server-Struktur) einzusetzen, ohne dass hierfür separate Quellcodestämme und/oder eine Neukompilierung der Applikation erforderlich wären.

Die von dem Framework zur Verfügung gestellten Funktionen sind in einer Anzahl von Komponenten strukturiert, die - wie vorstehend eingeführt - nach Maßgabe ihrer Schichtzugehörigkeit auch als Servicekomponenten, Präsentationskomponenten, Toolkits, etc. bezeichnet werden.

Die Servicekomponenten (inklusive der Komponenten der Web-Serviceschicht 6 unterscheiden sich hinsichtlich ihrer programmiertechnischen und ablauftechnischen Implementierung grundsätzlich von den Komponenten der Toolkit-Schicht 4 und der Präsentationsschicht 5.

So sind die Servicekomponenten multi-thread-fähig und einfach instanziierbar implementiert, während die Komponenten der Toolkit-Schicht 4 und Präsentationsschicht 5 single-thread-fähig und mehrfach instanziierbar ausgebildet sind. Zu den Vorteilen dieser schichtabhängig gegensätzlichen Implementierung der Servicekomponenten und Toolkits wird wiederum auf die vorstehenden Ausführungen verwiesen.

Jedes der nachfolgend beschriebenen Frameworks ist allein mit den Komponenten der Serviceschicht 3 und Toolkit-Schicht 4, sowie ferner den Komponenten der Präsentationsschicht 5 für den Offline-Betrieb vollständig funktionsfähig.

Die Web-Serviceschicht 6 unterstützt den Online-Betrieb einer lokal installierten Applikation im Rahmen eines medizinischen Netzwerkes und ermöglicht den Zugriff auf Dienste, die entfernt auf anderen Knoten des Netzwerkes lokalisiert sind. Die Trennung von Serviceschicht 3 und Web-Serviceschicht 6 ermöglicht auf einfache Weise die Realisierung von Applikationen, die sowohl für den Online-Betrieb als auch Offline-Betrieb als auch für einen unproblematischen Wechsel zwischen beiden Betriebszuständen zur Laufzeit tauglich sind.

Zusätzlich zu der Gliederung des (bzw. jeden) Frameworks in die Schichten 3 bis 6 und unabhängig hiervon ist bezüglich des Frameworks eine Stabilitätsschicht 20 sowie eine Entwicklungsschicht 21 definiert. Hiervon nimmt die Stabilitätsschicht 20 einen hierarchisch untergeordneten Bereich, und die Entwicklungsschicht 21 den daran anschließenden hierarchisch übergeordneten Bereich des Frameworks ein. Im dargestellten Ausführungsbeispiel ist die Grenze zwischen der Stabilitätsschicht 20 und der Entwicklungsschicht 21 derart gewählt, dass die Stabilitätsschicht 20 genau die Serviceschicht 3 und die Web-Serviceschicht 6 umfasst, während die Entwicklungsschicht 21 die Toolkit-Schicht 4 und die Präsentationsschicht 5 umfasst. Grundsätzlich kann die Grenze zwischen der Stabilitätsschicht 20 und der Entwicklungsschicht 21 aber unabhängig von den Schichten 3 bis 6 gewählt werden. Entsprechend ihrer Zugehörigkeit zu der Stabilitätsschicht 20 werden die Module der Serviceschicht 3 und Web-Serviceschicht 6 streng rückwärtskompatibel versioniert. Für die der Entwicklungsschicht 21 zugehörigen Module besteht dagegen kein Zwang zur rückwärtskompatiblen Versionierung, so dass diese Komponenten flexibel weiterentwickelt werden können.

In FIG 2 ist eine erste Ausführungsform dargestellt, in welcher das System 1 ein auf die Sub-Domain des Datenmanagement (kurz DM) ausgerichtetes Framework umfasst. Dieses Framework ist nachfolgend als DM-Framework 2 bezeichnet.

Im Einzelnen umfasst gemäß FIG 1 die Serviceschicht 3 für das DM-Framework 2 als Servicekomponenten ein Dateizugangsmodul 10, ein Informations-Modell-Abbildungsmodul 11, ein Arbeitsbereichsmodul 12, ein DM-Dienstmodul 13 sowie ein Standard-DM-Zugriffsmodul 14.

In der Toolkit-Schicht 4 umfasst das DM-Framework 2 eine einziges Toolkit, das nachfolgend als DM-Grundmodul 15 bezeichnet ist. Mit dem DM-Grundmodul 15 der Toolkit-Schicht 4 korrespondiert in der Präsentationsschicht 5 eine Präsentationskomponente, die als DM-Präsentationsmodul 16 bezeichnet ist.

Das DM-Grundmodul 15 ist innerhalb der Programmierumgebung 7 auf einer dem DM-Framework 2 zugeordneten Master-Seite 18 instanziierbar. Das DM-Präsentationsmodul 16 ist auf einer entsprechend innerhalb der Programmierumgebung 8 vorgesehenen Master-Seite 19 instanziierbar. Die Masterseiten 18 und 19 stehen untereinander im Datenaustausch.

Ferner enthält das DM-Framework 2 in der Web-Serviceschicht 6 eine als Remote-DM-Dienste 17 bezeichnete Komponente.

Die Komponenten des DM-Frameworks 2 weisen jeweils die bzw. alle diesbezüglich in dem der Figurenbeschreibung voranstehenden Beschreibungsteil genannten Funktionen auf. Insbesondere umfassen aber die in dem DM-Dienstmodul 13 implementierten Funktionen die vorstehend beschriebenen Sub-Systeme, nämlich das Indexing-Sub-System, das Query-Sub-System, das Synchronisation-Sub-System, das Data-Locking-Sub-System, das Security-Sub-System und das Work-Flag-Sub-System. Die Sub-Systeme sind in Form von Plugins realisiert und können zur Laufzeit einer Applikation wahlweise einzeln oder in beliebiger Kombination für die Ausführung im Rahmen der Applikation aktiviert oder deaktiviert werden.

Die in der Web-Serviceschicht 6, der Serviceschicht 3 und der Toolkit-Schicht 4 enthaltenen Komponenten sind zueinander in einer strengen Hierarchie angeordnet, die sich darin äußert, dass jede der Komponenten ausschließlich auf hierarchisch untergeordnete Komponenten zugreifen kann, während ein Zugriff von einer der Komponenten auf eine neben- oder übergeordnete Komponente ausgeschlossen ist.

Die zwischen den Komponenten vorgesehenen Abhängigkeiten, d.h. Zugriffsmöglichkeiten sind in FIG 1 durch Pfeile schematisch angedeutet. Danach wird auf die in der Hierarchie zuunterst angeordneten Remote-DM-Dienste 17 von dem direkt übergeordneten Dateizugangsmodul 10 aus zugegriffen. Auf jede der Module 10 und 11 kann sowohl von dem Arbeitsbereichsmodul 12, dem DM-Dienstmodul 13 als auch dem Standard-DM-Zugriffsmodul 14 aus zugegriffen werden. Innerhalb der Serviceschicht 3 ist hierbei das Standard-DM-Zugriffsmodul 14 hierarchisch zuoberst angeordnet und seinerseits dazu ausgebildet, auf das DM-Dienstmodul 13 zuzugreifen. Zwischen dem DM-Dienstmodul 13 und dem Arbeitsbereichsmodul 12 bzw. zwischen dem Standard-DM-Zugriffsmodul 14 und dem Arbeitsbereichsmodul 12 ist keine direkte Zugriffsmöglichkeit vorgesehen.

Über das in der Toolkit-Schicht 4 angeordnete DM-Grundmodul 15 ist eine Zugriffsmöglichkeit sowohl auf das Standard-DM-Zugriffsmodul 14 als auch auf das DM-Dienstmodul 13 und das Arbeitsbereichsmodul 12 vorgesehen.

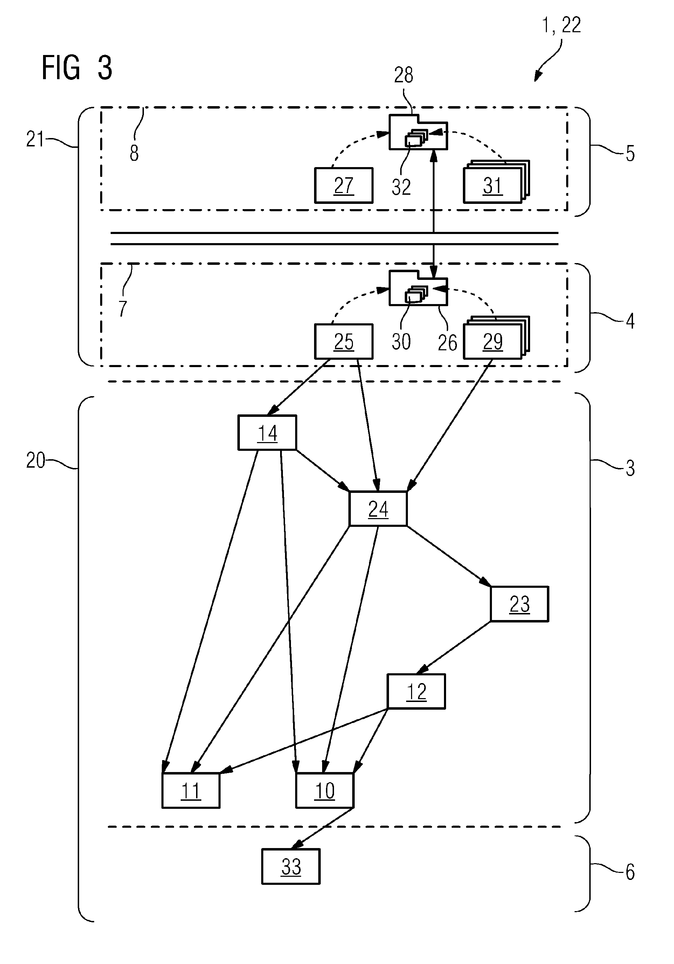

In der in FIG 3 dargestellten Variante des Systems 1 umfasst dieses ein auf die Sub-Domain des (Daten-)Transfer (TF) ausgerichtetes Framework, das nachfolgend als TF-Framework 22 bezeichnet ist.

Analog zu dem DM-Framework 2 umfasst das TF-Framework 22 in der Serviceschicht 3 das Dateizugangsmodul 10, das Informations-Modell-Abbildungsmodul 11, das Arbeitsbereichsmodul 12 und das Standard-DM-Zugriffsmodul 14. Das TF-Framework 22 umfasst weiterhin in der Serviceschicht 3 ein Pipeline-Modul 23 und ein TF-Dienstmodul 24, wobei das Pipeline-Modul 23 dem Arbeitsbereichsmodul 12 übergeordnet ist und auf letzteres zugreift. Das TF-Dienstmodul 24 ist wiederum dem Pipeline-Modul 23 übergeordnet und greift auf Funktionen des Pipeline-Moduls 23, des Dateizugangsmoduls 10 und des Informations-Modell-Abbildungsmoduls 11 zu. Im Rahmen des TF-Frameworks 22 greift das Standard-DM-Zugriffsmodul 10 auf Funktionen des Dateizugangsmoduls 10, des Informations-Modell-Abbildungsmoduls 11 sowie - abweichend von FIG 1 - auf Funktionen des TF-Dienstmoduls 24 zu.

Als Komponenten der Toolkit-Schicht 4 umfasst das TF-Framework 22 ein TF-Grundmodul 25, das auf einer zugehörigen Master-Seite 26 der Programmierumgebung 7 instanziierbar ist. Mit dem TF-Grundmodul 25 korrespondiert innerhalb der Präsentationsschicht 5 ein TF-Präsentationsmodul 27, das auf einer entsprechenden Master-Seite 28 der Programmierumgebung 8 instanziierbar ist.

In der Toolkit-Schicht 4 umfasst das TF-Framework 22 als weitere Komponenten eine Anzahl von TF-Baukastenmodulen 29, deren jedes einer aus der Masterseite 26 ableitbaren Inhaltsseite 30 der Programmierumgebung 7 als individueller Inhalt zuordenbar ist. Mit den TF-Baukastenmodulen 29 korrespondieren entsprechende TF-Baukastenmodule 31 der Präsentationsschicht 5, die entsprechenden Inhaltsseiten 32 der Programmierumgebung 8 zuordenbar sind.

Die Masterseiten 26,28 und Inhaltsseiten 30, 32 stehen untereinander im Datenaustausch.

Ausgehend von dem TF-Grundmodul 25 ist eine direkte Zugriffsmöglichkeit auf Funktionen des TF-Dienstmoduls 24 sowie des Standard-DM-Zugriffsmoduls 14 vorgesehen. Ausgehend von den TF-Baukastenmodulen 29 ist eine Zugriffsmöglichkeit auf das TF-Dienstmodul 24 vorgesehen.

Als Komponente der Web-Serviceschicht 3 weist das TF-Framework 22 Remote-TF-Dienste 33 auf, auf die von dem Dateizugangsmodul 10 aus zugreifbar ist.

Die Komponenten des TF-Frameworks 22 weisen wiederum jeweils die bzw. alle diesbezüglich in dem der Figurenbeschreibung voranstehenden Beschreibungsteil genannten Funktionen auf.

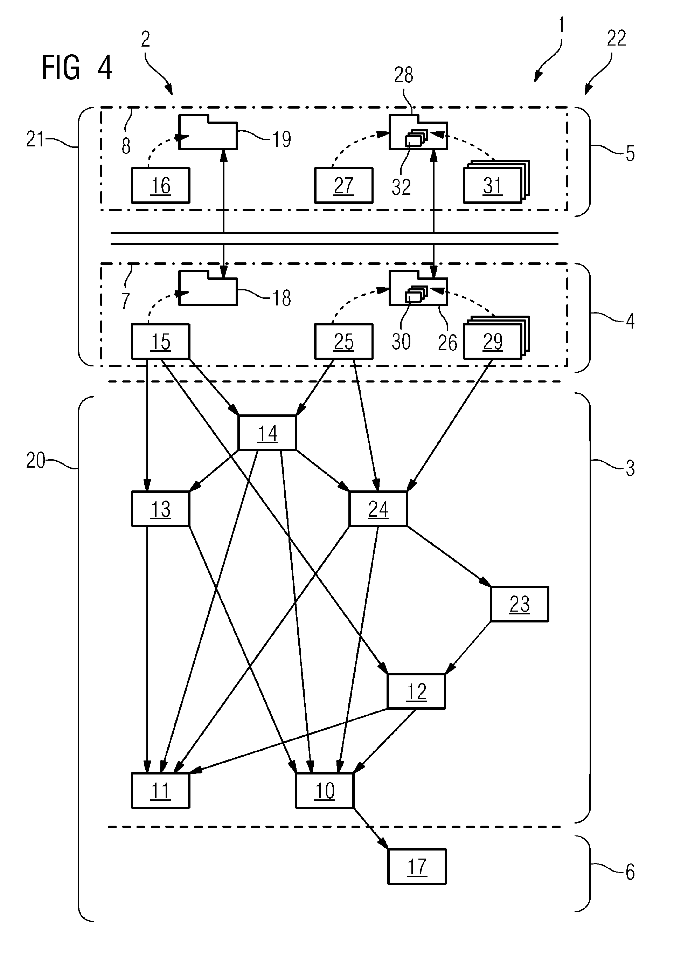

In der in FIG 4 dargestellten Variante des Systems 1 weist dieses das DM-Framework 2 und das TF-Framework 22 in Kombination auf.

In dieser Ausführung sind ausgehend von dem Standard-DM-Zugriffsmodul 14 Zugriffsmöglichkeiten sowohl auf das DM-Dienstmodul 13 als auch auf das TF-Dienstmodul 24, sowie ferner auf das Dateizugangsmodul 10 und das Informations-Modell-Abbildungsmodul 11 vorgesehen. Das Standard-DM-Zugriffsmodul 14 verfügt in dieser Ausführung insbesondere auch über Funktionen, die einen kombinierten Zugriff auf Funktionen der Dienstmodule 13 und 24 ermöglichen.

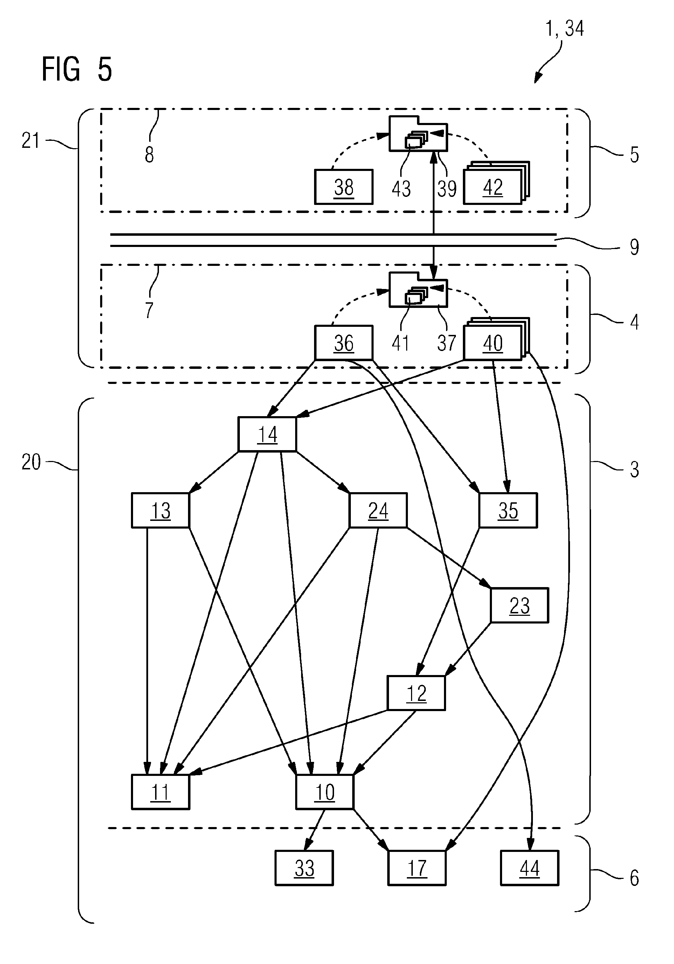

In der in FIG 5 gezeigten Variante des Systems 1 umfasst dieses ein für die Sub-Domain des Aufgabenlisten- bzw. Worklist-Management konzipiertes Framework, das nachfolgend kurz als WL-Framework 34 bezeichnet ist.

Analog zu der vorstehend Variante des Systems 1 umfasst das WL-Framework 34 die Service- und Web-Servicekomponenten des DM-Frameworks 2 und des TF-Frameworks 22, nämlich die Remote-DM-Dienste 17 und die Remote-TF-Dienste 33, das Dateizugangsmodul 10, das Informations-Modell-Abbildungsmodul 11, das Arbeitsbereichsmodul 12, das Pipeline-Modul 23, das DM-Dienstmodul 13, das TF-Dienstmodul 24 und das Standard-DM-Zugriffsmodul 14.

Zusätzlich zu diesen Komponenten umfasst das WL-Framework 34 in der Serviceschicht 3 ein WL-Dienstmodul 35, das dem Arbeitsplatzmodul 12 übergeordnet ist und auf dessen Funktionalität zugreift.

In der Toolkit-Schicht 4 umfasst das WL-Framework 34 ein WL-Grundmodul 36, das insbesondere auf einer entsprechenden Master-Seite 37 der Programmierumgebung 7 instanziierbar ist. Mit dem WL-Grundmodul 36 korrespondiert in der Präsentationsschicht 5 ein WL-Präsentationsmodul 38, das wiederum auf einer entsprechenden Master-Seite 39 der Programmierumgebung 8 instanziierbar ist.

Das WL-Grundmodul 36 greift hierbei einerseits auf das WL-Dienstmodul 35 zu. Andererseits greift das WL-Grundmodul 36 über das Standard-DM-Zugriffsmodul 14 auf Datenmanagement-und Transferfunktionalitäten zu.

In der Toolkit-Schicht 4 umfasst das WL-Framework 34 als weitere Komponenten optional eine Anzahl von kundenspezifisch erstellbaren WL-Baukastenmodulen 40, deren jedes einer aus der Masterseite 37 ableitbaren Inhaltsseite 41 der Programmierumgebung 7 als individueller Inhalt zuordenbar ist. Mit den WL-Baukastenmodulen 40 korrespondieren entsprechende WL-Baukastenmodule 42 der Präsentationsschicht 5, die entsprechenden Inhaltsseiten 43 der Programmierumgebung 8 zuordenbar sind. Die WL-Baukastenmodule 40 greifen hierbei beispielhaft auf das WL-Dienstmodul 35, das Standard-DM-Zugriffsmodul 14 und die Remote-DM-Dienste 17 zu.

Die Masterseiten 37, 39 und Inhaltsseiten 41, 43 stehen untereinander im Datenaustausch.

Als subdomain-spezifische Komponente der Web-Serviceschicht 6 umfasst das WL-Framework 34 Remote-WL-Dienste 44, auf die direkt von dem WL-Grundmodul 36 aus zugreifbar ist.

Die Komponenten des WL-Frameworks 34 weisen wiederum jeweils die bzw. alle diesbezüglich in dem der Figurenbeschreibung voranstehenden Beschreibungsteil genannten Funktionen auf.

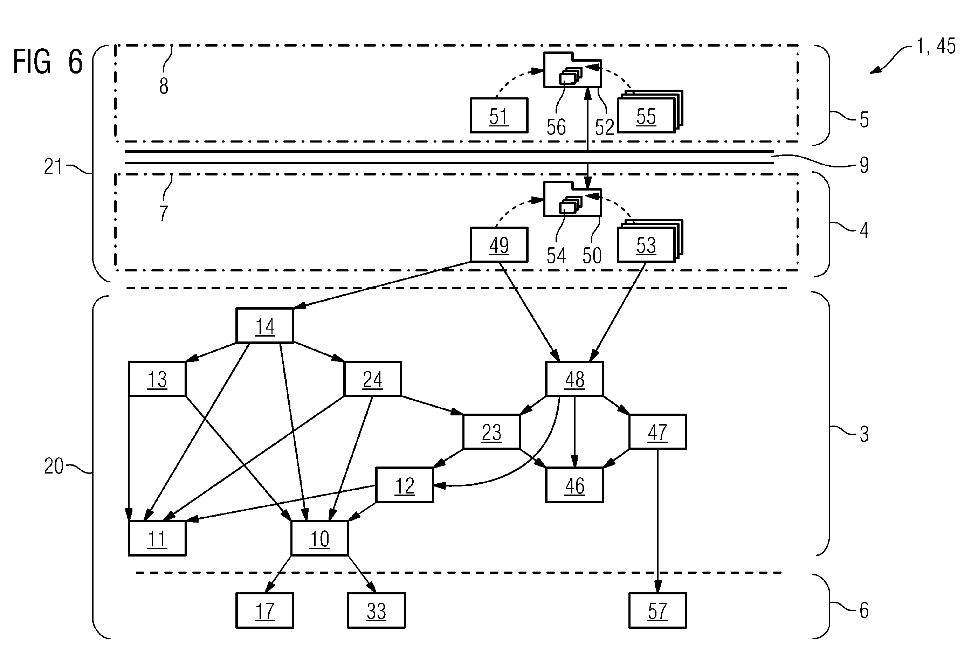

In der in FIG 6 gezeigten Variante des Systems 1 umfasst dieses ein für die Sub-Domain der Bildbearbeitung konzipiertes Framework, das nachfolgend kurz als IP-Framework 45 bezeichnet ist.

Analog zu der vorstehend Variante des Systems 1 umfasst das IP-Framework 45 die Service- und Web-Servicekomponenten des DM-Frameworks 2 und des TF-Frameworks 22, nämlich die Remote-DM-Dienste 17 und die Remote-TF-Dienste 33, sowie das Dateizugangsmodul 10, das Informations-Modell-Abbildungsmodul 11, das Arbeitsbereichsmodul 12, das Pipeline-Modul 23, das DM-Dienstmodul 13, das TF-Dienstmodul 24 und das Standard-DM-Zugriffsmodul 14.

Zusätzlich zu diesen Komponenten umfasst das IP-Framework 45 in der Serviceschicht 3 ein dem Pipeline-Modul 23 untergeordnetes Segmentierungsmodul 46, ein dem Pipeline-Modul 23 nebengeordnetes Graphik/Anzeige-Modul 47 und ein dem Pipeline-Modul 23 übergeordnetes IP-Dienstmodul 48. Sowohl das Pipeline-Modul 23 als auch das Graphik/Anzeige-Modul 47 als auch das IP-Dienstmodul 48 greifen hierbei auf das Segmentierungsmodul 46 zu. Ausgehend von dem IP-Dienstmodul 48 sind zusätzlich direkte Zugriffsmöglichkeiten auf das Pipeline-Modul 23, das Graphik/Anzeige-Modul 47 sowie auf das Arbeitsbereichsmodul 12 vorgesehen.

In der Toolkit-Schicht 4 umfasst das IP-Framework 45 ein Bildanzeige-Grundmodul 49, das insbesondere auf einer entsprechenden Master-Seite 50 der Programmierumgebung 7 instanziierbar ist. Mit dem IP-Grundmodul 49 korrespondiert in der Präsentationsschicht 5 ein Bildanzeige-Präsentationsmodul 51, das wiederum auf einer entsprechenden Master-Seite 52 der Programmierumgebung 8 instanziierbar ist.

Das IP-Grundmodul 49 greift hierbei einerseits auf das IP-Dienstmodul 48 zu. Andererseits greift das IP-Grundmodul 49 über das Standard-DM-Zugriffsmodul 14 auf Datenmanagement-und Transferfunktionalitäten zu.

In der Toolkit-Schicht 4 umfasst das IP-Framework 45 als weitere Komponenten optional eine Anzahl von kundenspezifisch erstellbaren Bildbearbeitungs(IP)-Baukastenmodulen 53, deren jedes einer aus der Masterseite 50 ableitbaren Inhaltsseite 54 der Programmierumgebung 7 als individueller Inhalt zuordenbar ist. Mit den IP-Baukastenmodulen 53 korrespondieren entsprechende IP-Baukastenmodule 55 der Präsentationsschicht 5, die entsprechenden Inhaltsseiten 56 der Programmierumgebung 8 zuordenbar sind. Die IP-Baukastenmodule 53 greifen hierbei beispielsweise auf das IP-Dienstmodul 48 zu.

Als subdomain-spezifische Komponente der Web-Serviceschicht 6 umfasst das IP-Framework 45 Remote-Display-Dienste 57, auf die von dem Graphik/Anzeige-Modul 47 aus zugreifbar ist.

Die Komponenten des IP-Frameworks 45 weisen wiederum jeweils die bzw. alle diesbezüglich in dem der Figurenbeschreibung voranstehenden Beschreibungsteil genannten Funktionen auf.

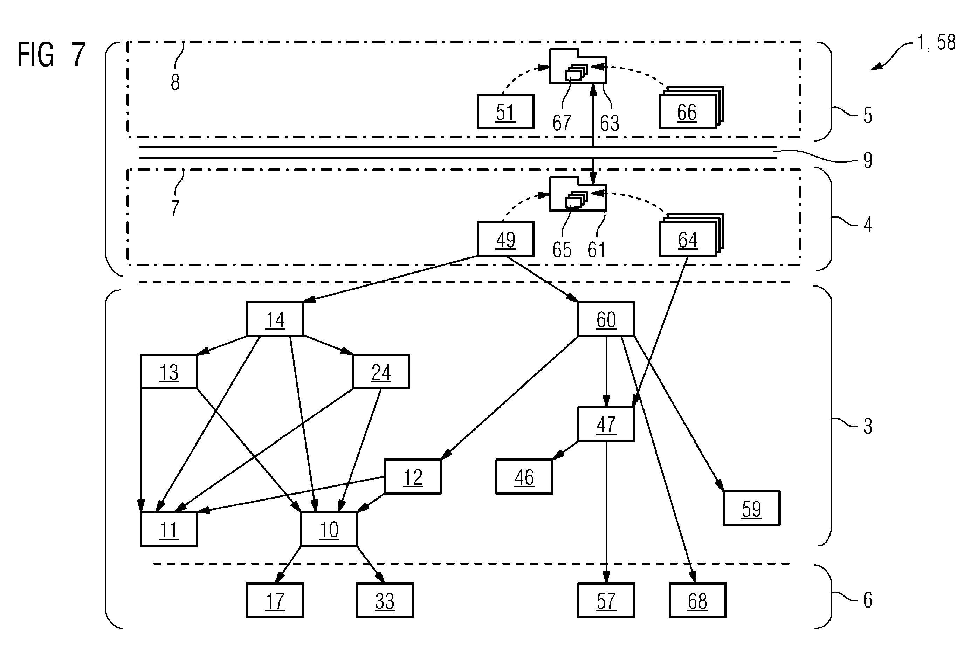

In der in FIG 7 gezeigten Variante des Systems 1 umfasst dieses ein für die Sub-Domain der Volumenbearbeitung konzipiertes Framework, das nachfolgend kurz als VOL-Framework 58 bezeichnet ist.

Das VOL-Framework 58 umfasst die Service- und Web-Servicekomponenten des DM-Frameworks 2, sowie teilweise Service- und Web-Servicekomponenten des TF-Frameworks 22 und des IP-Frameworks 45, nämlich die Remote-DM-Dienste 17, die Remote-TF-Dienste 33 und Remote-Display-Dienste 57, sowie das Dateizugangsmodul 10, das Informations-Modell-Abbildungsmodul 11, das Arbeitsbereichsmodul 12, das DM-Dienstmodul 13, das TF-Dienstmodul 24 und das Standard-DM-Zugriffsmodul 14, das Segmentierungsmodul 46 und das Graphik/Anzeige-Modul 47. Das im Rahmen des TF-Frameworks 22 und des IP-Frameworks 45 vorgesehene Pipeline-Modul 23 ist in der Ausführung des Systems 1 gemäß FIG 7 dagegen nicht vorgesehen, zumal diese Komponente auf Modifikation von Bilddaten spezifiziert ist, und für den Zweck der reinen Volumendatenbearbeitung nicht benötigt wird. Auf die spezifischen Service- und Web-Servicekomponenten des IP-Frameworks 45, nämlich das Segmentierungsmodul 46 und das Graphik/Anzeige-Modul 47 wird im Rahmen des VOL-Frameworks 58 für den Zweck der graphischen Anzeige der Volumendaten zurückgegriffen, zumal auch Volumendaten auf einem Bildschirm oder Ausdruck notwendigerweise lediglich in reduzierter zweidimensionaler Form, mithin also in Form von Bilddaten, darstellbar sind.

Zusätzlich zu den genannten Komponenten umfasst das VOL-Framework 58 in der Serviceschicht 3 ein 3D-Bildsynthesemodul 59 und ein VOL-Dienstmodul 60.

Auch in der Toolkit-Schicht 4 nutzt das VOL-Framework 58 mit dem Bildanzeige-Grundmodul 49 eine spezifische Komponente des IP-Frameworks 45 mit. Dieses Bildanzeige-Grundmodul 49 ist aber im Rahmen des VOL-Frameworks 58 auf einer eigenen Master-Seite 61 der Programmierumgebung 7 instanziierbar. Ebenso umfasst das VOL-Framework 58 in der Präsentationsschicht 5 das korrespondierende Bildanzeige-Präsentationsmodul 51, das wiederum auf einer für das VOL-Framework 58 spezifischen Master-Seite 63 der Programmierumgebung 8 instanziierbar ist.

Das Bildanzeige-Grundmodul 49 greift hierbei auf das Standard-DM-Zugriffsmodul 14 sowie - abweichend von der Ausführung gemäß FIG 6 - auf das VOL-Dienstmodul 60 zu.

In der Toolkit-Schicht 4 umfasst das VOL-Framework 58 als weitere Komponenten optional eine Anzahl von für die Volumenbearbeitung spezifischen VOL-Baukastenmodulen 64, deren jedes einer aus der Masterseite 61 ableitbaren Inhaltsseite 65 der Programmierumgebung 7 als individueller Inhalt zuordenbar ist. Mit den VOL-Baukastenmodulen 64 korrespondieren entsprechende VOL-Baukastenmodule 66 der Präsentationsschicht 5, die entsprechenden Inhaltsseiten 67 der Programmierumgebung 8 zuordenbar sind. Die VOL-Baukastenmodule 64 greifen hierbei beispielsweise auf das Graphik/Anzeige-Modul 47 zu.

Als subdomain-spezifische Komponente der Web-Serviceschicht 6 umfasst das VOL-Framework 58 VOL-Remote-Dienste 68, auf die von dem VOL-Dienstmodul 60 aus zugreifbar ist.

Die Komponenten des VOL-Frameworks 58 weisen wiederum jeweils die bzw. alle diesbezüglich in dem der Figurenbeschreibung voranstehenden Beschreibungsteil genannten Funktionen auf.

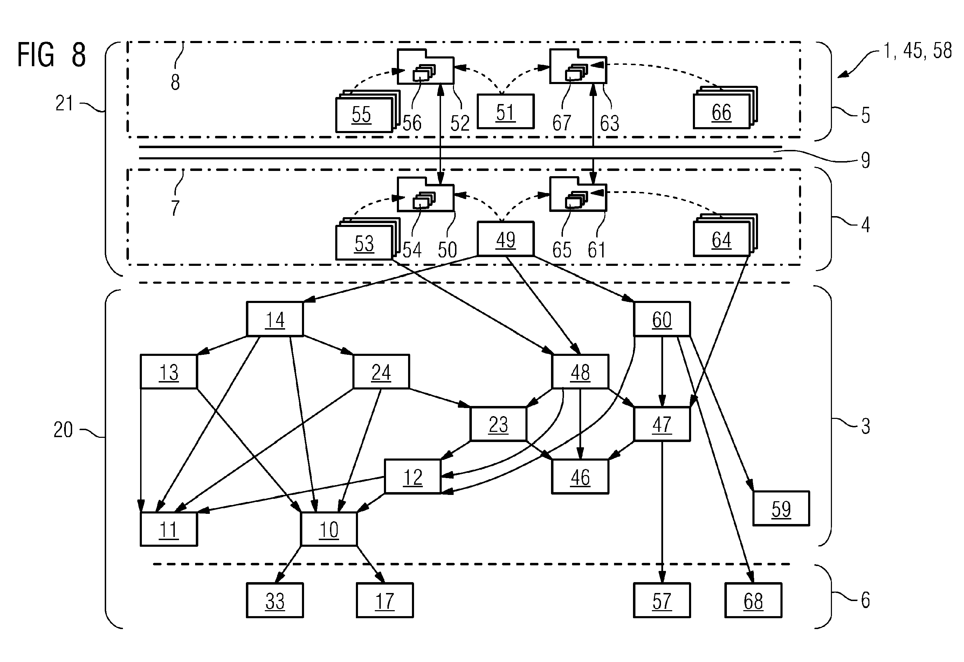

Aufgrund der engen inhaltlichen und strukturellen Verknüpfung des IP-Frameworks 45 mit dem VOL-Framework 58 werden beide Frameworks bevorzugt in Kombination miteinander eingesetzt. Eine entsprechende Ausführung des Systems 1 ist in FIG 8 dargestellt.

In dieser Ausführung greift das beiden Frameworks 45,58 gemeinsame Bildanzeige-Grundmodul 49 auf das Standard-DM-Zugriffsmodul 14 sowie - abweichend von den voranstehend beschriebenen Ausführungen - sowohl auf das IP-Dienstmodul 48 als auch auf das VOL-Dienstmodul 60 zu.

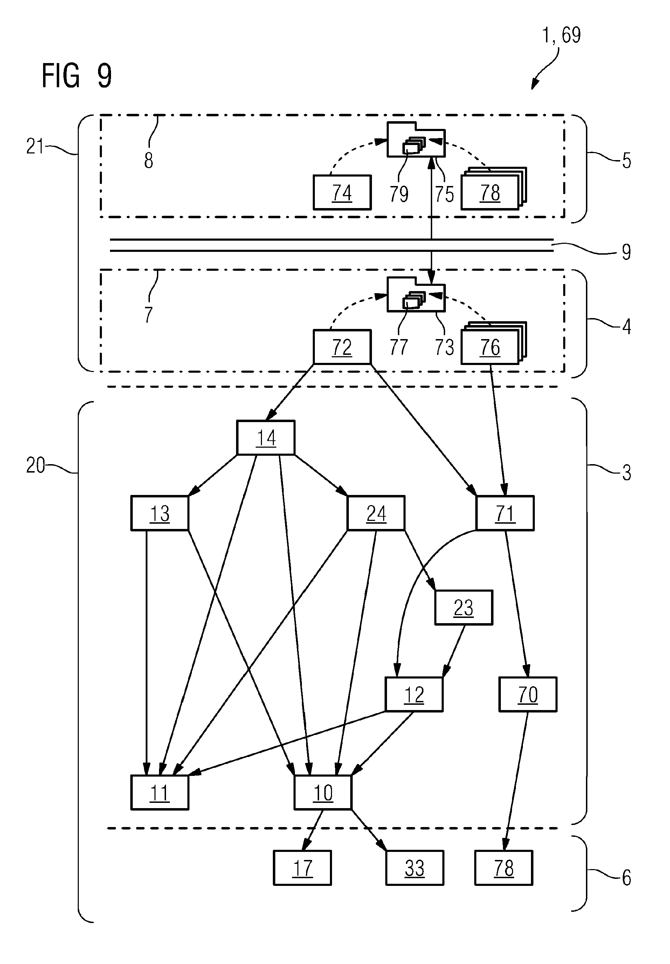

In der in FIG 9 gezeigten Variante des Systems 1 umfasst dieses ein für die Sub-Domain der Berichterstellung konzipiertes Framework, das nachfolgend kurz als REP-Framework 69 bezeichnet ist.

Auch das REP-Framework 69 umfasst die Service- und Web-Servicekomponenten des DM-Frameworks 2 und des TF-Frameworks 22, nämlich die Remote-DM-Dienste 17 und die Remote-TF-Dienste 33, sowie das Dateizugangsmodul 10, das Informations-Modell-Abbildungsmodul 11, das Arbeitsbereichsmodul 12, das Pipeline-Modul 23, das DM-Dienstmodul 13, das TF-Dienstmodul 24 und das Standard-DM-Zugriffsmodul 14.

Zusätzlich zu diesen Komponenten umfasst das REP-Framework 69 in der Serviceschicht 3 ein Terminologiemodul 70 sowie ein diesem und dem Arbeitsplatzmodul 12 übergeordnetes REP-Dienstmodul 71, das auf die Funktionalität der beiden erstgenannten Module 70 und 71 zugreift.

In der Toolkit-Schicht 4 umfasst das REP-Framework 69 ein REP-Grundmodul 72, das insbesondere auf einer entsprechenden Master-Seite 73 der Programmierumgebung 7 instanziierbar ist. Mit dem REP-Grundmodul 72 korrespondiert in der Präsentationsschicht 5 ein REP-Präsentationsmodul 74, das wiederum auf einer entsprechenden Master-Seite 75 der Programmierumgebung 8 instanziierbar ist.

Das REP-Grundmodul 72 greift hierbei einerseits auf das REP-Dienstmodul 71 zu. Andererseits greift das REP-Grundmodul 72 über das Standard-DM-Zugriffsmodul 14 auf Datenmanagement-und Transferfunktionalitäten zu.

In der Toolkit-Schicht 4 umfasst das REP-Framework 69 als weitere Komponenten optional eine Anzahl von kundenspezifisch erstellbaren REP-Baukastenmodulen 76, deren jedes einer aus der Masterseite 73 ableitbaren Inhaltsseite 77 der Programmierumgebung 7 als individueller Inhalt zuordenbar ist. Mit den REP-Baukastenmodulen 76 korrespondieren entsprechende REP-Baukastenmodule 78 der Präsentationsschicht 5, die entsprechenden Inhaltsseiten 79 der Programmierumgebung 8 zuordenbar sind. Die REP-Baukastenmodule 76 greifen hierbei beispielsweise auf das REP-Dienstmodul 71 zu.

In der Web-Serviceschicht 6 umfasst das REP-Framework 69 zusätzlich eine als Zentral-Terminologie-Service 80 bezeichnete Komponente auf, auf die das Terminologiemodul 70 im Online-Betrieb zugreift.

Die Komponenten des REP-Frameworks 69 weisen wiederum jeweils die bzw. alle diesbezüglich in dem der Figurenbeschreibung voranstehenden Beschreibungsteil genannten Funktionen auf.

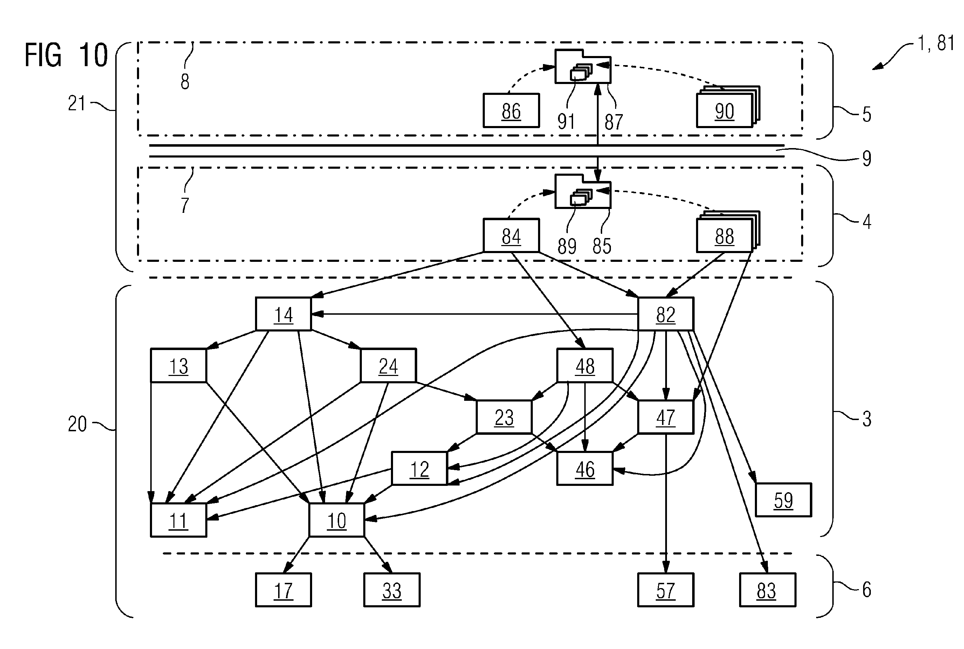

In der in FIG 10 gezeigten Variante des Systems 1 umfasst dieses ein für die Sub-Domain der Bilderfassung (bzw. Untersuchung / Examination) konzipiertes Framework, das nachfolgend kurz als EXAM-Framework 81 bezeichnet ist.

Das EXAM-Framework 81 umfasst die Service- und Web-Servicekomponenten des DM-Frameworks 2 und des TF-Frameworks 22, nämlich die Remote-DM-Dienste 17 und die Remote-TF-Dienste 33, sowie das Dateizugangsmodul 10, das Informations-Modell-Abbildungsmodul 11, das Arbeitsbereichsmodul 12, das Pipeline-Modul 23, das DM-Dienstmodul 13, das TF-Dienstmodul 24 und das Standard-DM-Zugriffsmodul 14.

Das EXAM-Framework 81 umfasst weiter die Service- und Web-Servicekomponenten des IP-Frameworks 45, sowie Teile des VOL-Frameworks 58, nämlich das Segmentierungsmodul 46, das Graphik/Anzeige-Modul 47 und das IP-Dienstmodul 48 sowie die Remote-Display-Dienste 57 und das 3D-Bildsynthese-Modul 59.

Das EXAM-Framework 81 umfasst weiter in der Serviceschicht 3 ein EXAM-Dienstmodul 82. Ausgehend von dem EXAM-Dienstmodul 82 sind direkte Zugriffsmöglichkeiten auf das Dateizugangsmodul 10, das Informations-Modell-Abbildungsmodul 11, das Arbeitsbereichsmodul 12, das Segmentierungsmodul 46, das Graphik/Anzeige-Modul 47 sowie das 3D-Bildsynthesemodul 59 vorgesehen. Das EXAM-Dienstmodul 82 ist hierbei auch dem Standard-DM-Zugriffsmodul 14 übergeordnet, und greift auf dieses zu.

Als subdomain-spezifische Komponente der Web-Serviceschicht 6 umfasst das EXAM-Framework 81 Remote-EXAM-Dienste 83, auf die ebenfalls von dem EXAM-Dienstmodul 82 aus zugreifbar ist.

In der Toolkit-Schicht 4 umfasst das EXAM-Framework 81 ein EXAM-Grundmodul 84, das insbesondere auf einer entsprechenden Master-Seite 85 der Programmierumgebung 7 instanziierbar ist. Mit dem EXAM-Grundmodul 84 korrespondiert in der Präsentationsschicht 5 ein EXAM-Präsentationsmodul 86, das wiederum auf einer entsprechenden Master-Seite 87 der Programmierumgebung 8 instanziierbar ist.

In der Toolkit-Schicht 4 umfasst das EXAM-Framework 81 als weitere Komponenten optional eine Anzahl von kundenspezifisch erstellbaren EXAM-Baukastenmodulen 88, deren jedes einer aus der Masterseite 85 ableitbaren Inhaltsseite 89 der Programmierumgebung 7 als individueller Inhalt zuordenbar ist. Mit den EXAM-Baukastenmodulen 88 korrespondieren entsprechende EXAM-Baukastenmodule 90 der Präsentationsschicht 5, die entsprechenden Inhaltsseiten 91 der Programmierumgebung 8 zuordenbar sind. Die EXAM-Baukastenmodule 88 greifen hierbei beispielsweise auf das EXAM-Dienstmodul 82 zu.

Die Komponenten des EXAM-Frameworks 81 weisen wiederum jeweils die bzw. alle diesbezüglich in dem der Figurenbeschreibung voranstehenden Beschreibungsteil genannten Funktionen auf. Insbesondere umfasst der Funktionsumfang des EXAM-Dienstmoduls 82 die vorstehend genannten Sub-Systeme, nämlich das Bildquellen-Kontroll-Sub-System und das Rohdaten-Anpassungs-Sub-System.

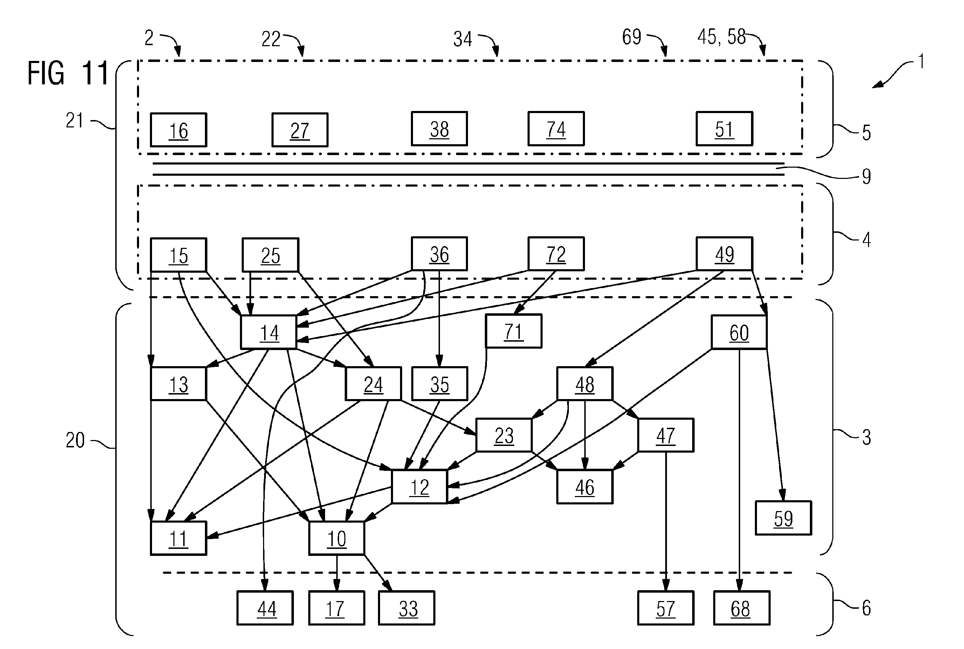

Die in FIG 11 dargestellte Variante des Systems 1 umfasst in Kombination das DM-Framework 2, das TF-Framework 22, das WL-Framework 34, das IP-Framework 45, das VOL-Framework 58 und das REP-Framework 69, wobei jedes der Frameworks 2,22,34, 45,58 und 69 in der vorstehend beschriebenen Weise aufgebaut ist (Die framework-spezifischen Baukastenmodule, sowie die Master- und Inhaltsseiten der Programmierungsgebungen 7 und 8 sind lediglich aus Gründen der Übersichtlichkeit nicht explizit dargestellt). Eine weitere (nicht näher dargestellte) Variante des Systems 1 umfasst zusätzlich das EXAM-Framework 81.

## Patentansprüche

1. System (1) zur Erzeugung und zum Betrieb einer Softwareapplikation für die medizinische Bildgebung, mit mindestens einem Framework (2,22,34,45,58,69,81), das eine Serviceschicht (3) sowie eine dieser als Applikations-Programmier-Schnittstelle übergeordnete Toolkit-Schicht (4) aufweist, wobei Funktionen der Toolkit-Schicht (4) und der Serviceschicht (3) in jeweils einer Anzahl von Komponenten zusammengefasst sind, die streng hierarchisch angeordnet sind derart, dass auf eine beliebige Komponente stets ausschließlich von einer übergeordneten Komponente aus zugegriffen werden kann.

2. System (1) nach Anspruch 1,
wobei die oder jede Komponente der Serviceschicht (3) einfach-instanziierbar und multi-thread-fähig ausgebildet ist, während die oder jede Komponente der Toolkit-Schicht (4) mehrfach-instanzliierbar und single-thread-fähig ausgebildet ist.

3. System (1) nach Anspruch 1 oder 2,
wobei die oder jede Komponente der Toolkit-Schicht (4) für eine schnelle Applikationsentwicklung auf einer der Toolkit-Schicht (4) zugeordneten visuellen Programmierumgebung (7) instanziierbar und verknüpfbar sind.

4. System (1) nach einem der Ansprüche 1 bis 3,
wobei das oder jedes Framework (2,22,34,45,58,69,81) als Benutzerschnittstelle eine mit der Toolkit-Schicht (4) korrespondierende Präsentationsschicht (5) aufweist, innerhalb welcher zu mindestens einer Komponente der Toolkit-Schicht (4) eine zugeordnete Komponente vorgesehen ist.

5. System (1) nach Anspruch 4,
wobei die oder jede Komponente der Präsentationsschicht (5) für eine schnelle Applikationsentwicklung auf einer der Präsentationsschicht (5) zugeordneten visuellen Programmierumgebung (8) instanziierbar und verknüpfbar ist.

6. System (1) nach Anspruch 3 oder 5,
wobei auf der Programmierumgebung (7,8) pro Framework (2,22,34,45,58,69,81) eine eine Vorlage bildende Master-Seite (18,19,26,28,37,39,50,52,61,63,73,75,85,87) erzeugbar ist, der innerhalb des zugeordneten Frameworks (18,19,26,28,37,39,50,52,61,63,73,75,85,87) eine Komponente der Toolkit-Schicht (4) bzw. Präsentationsschicht (5) zuordenbar ist.

7. System (1) nach Anspruch 6,
wobei auf der Programmierumgebung (7,8) für zumindest ein Framework (22,34,45,58) mindestens eine aus der Master-Seite (26,28,37,39,50,52,61,63) als Vorlage abgeleitete Inhalts-Seite (30, 32, 41, 43, 54, 56, 65, 67, 76, 79, 88, 91) erzeugbar ist, der innerhalb des betreffenden Frameworks (22,34,45,58) mindestens eine Komponente der Toolkit-Schicht (4) als Inhalt zuordenbar ist.

8. System (1) nach einem der Ansprüche 1 bis 7,
wobei der Funktionsumfang mindestens einer Komponente der Serviceschicht durch mindestens ein Plugin reversibel erweiterbar ist.

9. System (1) nach Anspruch 8,
das das oder jedes Plugin zur Laufzeit aktivierbar und deaktivierbar ist.

10. System (1) nach einem der Ansprüche 1 bis 9,
wobei jede Komponente entsprechend ihrer hierarchischen Anordnung entweder einer Stabilitätsschicht (20) oder einer dieser übergeordneten Entwicklungsschicht (21) zugeordnet ist, wobei jede Komponente der Stabilitätsschicht (20) im Gegensatz zu der oder jeden Komponente der Entwicklungsschicht (21) streng rückwärtskompatibel versioniert ist.

11. System (1) nach Anspruch 10,
wobei die Grenze zwischen Stabilitätsschicht (20) und der Entwicklungsschicht (21) mit der Grenze zwischen der Serviceschicht (3) und der Toolkit-Schicht (4) zusammenfällt.

12. System (1) nach einem der Ansprüche 1 bis 11,
wobei die Komponenten des oder jedes Frameworks (2,22,34,45,58,69,81) plattformunabhängig in einer Laufzeitumgebung, insbesondere Microsoft .NET, ausführbar sind.

13. System (1) nach einem der Ansprüche 1 bis 12,
mit einem Datenmanagement-Framework (2) umfassend:
- als Komponenten der Serviceschicht (3)
- ein Dateizugangsmodul (10), das fundamentale Funktionen zum Lesen, Schreiben und Löschen von Daten sowie zum Erzeugen von Dateien in einem Dateisystem enthält,
- ein dem Dateizugangsmodul (10) übergeordnetes Datenmanagement-Dienstmodul (13), das Funktionen zur Datenauswahl sowie zur Dateizugangsregelung unter Zugriff auf das Dateizugangsmodul (10) enthält,
- als Komponente der Toolkit-Schicht (4) ein Datenmanagement-Grundmodul (15), das Funktionen zum Zugriff auf das Datenmanagement-Dienstmodul (13) im Rahmen einer Applikation enthält.

14. System (1) nach Anspruch 13,
wobei das Datenmanagement-Framework (2) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Datenmanagement-Dienstmodul (13) aus zugreifbar ist.

15. System (1) nach Anspruch 13 oder 14,
wobei das Datenmanagement-Framework (2) weiter umfasst:
als Komponente der Serviceschicht (3) ein dem Dateizugangsmodul (10), und gegebenenfalls dem Informations-Modell-Abbildungsmodul (11) übergeordnetes Arbeitsbereichsmodul (12), das Funktionen zur plattformunabhängigen lokalen Zwischenspeicherung von Daten unter Zugriff auf das Dateizugangsmodul (10) sowie gegebenenfalls auf das Informations-Modell-Abbildungsmodul (11) enthält, wobei auf das Arbeitsbereichsmodul (12) seinerseits direkt von dem Datenmanagement-Grundmodul (15) aus zugreifbar ist.

16. System (1) nach einem der Ansprüche 13 bis 15,
wobei das Datenmanagement-Framework (2) weiter umfasst:
als Komponente der Serviceschicht (3) ein dem Datenmanagement-Dienstmodul (13) übergeordnetes Standard-Datenmanagement-Zugriffsmodul (14), das Funktionen zum vereinfachten, insbesondere kombinierten Zugriff auf Funktionen des Datenmanagement-Dienstmoduls (13) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Datenmanagement-Grundmodul (15) aus zugreifbar ist.

17. System (1) nach einem der Ansprüche 13 bis 16,
wobei das Datenmanagement-Framework (2) weiter umfasst:
als Komponente einer der Serviceschicht (3) streng hierarchisch untergeordneten Web-Serviceschicht (6) Remote-Datenmanagement-Dienste (17), die Funktionen zum Lesen, Schreiben und Löschen von Daten sowie Erzeugen von Dateien auf einem entfernten Netzknoten enthalten, wobei auf die Remote-Datenmanagement-Dienste (17) von dem Dateizugangsmodul (10) aus zugreifbar ist.

18. System (1) nach einem der Ansprüche 13 bis 17,
wobei das Datenmanagement-Dienstmodul (13) ein Sub-System von Funktionen zur Indizierung von insbesondere lokal hinterlegten Dateien nach Maßgabe von diesen Dateien zugeordneten Meta-Daten enthält.

19. System (1) nach einem der Ansprüche 13 bis 18,
wobei das Datenmanagement-Dienstmodul (13) ein Sub-System von Funktionen zur Auswahl von Dateien durch Vergleich von diesen Dateien zugeordneten Meta-Daten mit vorgegebenen Suchbegriffen enthält.

20. System (1) nach einem der Ansprüche 13 bis 19,
wobei das Datenmanagement-Dienstmodul (13) ein Sub-System von Funktionen zur Synchronisierung von Dateien und/oder Vermeidung eines Mehrfachzugangs auf dieselbe Datei enthält.

21. System (1) nach einem der Ansprüche 13 bis 20,
wobei das Datenmanagement-Dienstmodul (13) ein Sub-System von Funktionen zur Kontrolle von benutzerspezifischen Zugangsrechten auf Dateien enthält.

22. System (1) nach einem der Ansprüche 13 bis 21,
wobei das Datenmanagement-Dienstmodul (13) ein Sub-System von Funktionen zur Verwaltung von Dateien zugeordneten Work-Flags enthält.

23. System (1) nach einem der Ansprüche 18 bis 22,
wobei mindestens ein Sub-System des Datenmanagement-Dienstmoduls (13) in Form eines oder mehrerer Plugins implementiert ist.

24. System (1) nach einem der Ansprüche 1 bis 23, mit einem Transfer-Framework (22) umfassend:
- als Komponenten der Serviceschicht (3)
- ein Dateizugangsmodul (10), das fundamentale Funktionen zum Lesen, Schreiben und Löschen von Daten sowie zum Erzeugen von Dateien in einem Dateisystem enthält,
- ein Pipeline-Modul (23), das eine Anzahl von einzeln oder in beliebiger Kombination zuschaltbaren Filtern zur Bearbeitung von Bilddaten definiert, sowie
- ein dem Dateizugangsmodul (10) und dem Pipeline-Modul (23) übergeordnetes Transfer-Dienstmodul (24), das Funktionen zur Übertragung von Daten zwischen zwei Knoten eines medizinischen Netzwerkes oder zwei Dateisystemen unter Zugriff auf das Dateizugangsmodul (10) und/oder das Pipeline-Modul (23) umfasst,
- als Komponente der Toolkit-Schicht (4) ein Transfer-Grundmodul (25), das Funktionen zum Zugriff auf das Transfer-Dienstmodul (24) im Rahmen einer Applikation enthält.

25. System (1) nach Anspruch 24,
wobei das Transfer-Framework (22) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Transfer-Dienstmodul (24) aus zugreifbar ist.

26. System (1) nach Anspruch 24 oder 25,
wobei das Transfer-Framework (22) weiter umfasst:
als Komponente der Serviceschicht (3) ein dem Dateizugangsmodul (10), und gegebenenfalls dem Informations-Modell-Abbildungsmodul (11) übergeordnetes Arbeitsbereichsmodul (12), das Funktionen zur plattformunabhängigen lokalen Zwischenspeicherung von Daten unter Zugriff auf das Dateizugangsmodul (10) sowie gegebenenfalls auf das Informations-Modell-Abbildungsmodul (11) enthält, wobei auf das Arbeitsbereichsmodul (12) seinerseits von dem Pipeline-Modul (23) aus zugreifbar ist.

27. System (1) nach einem der Ansprüche 24 bis 26,
wobei das Transfer-Framework (22) weiter umfasst:
als Komponente der Serviceschicht (3) ein dem Transfer-Dienstmodul (24) übergeordnetes Standard-Datenmanagement-Zugriffsmodul (14), das Funktionen zum vereinfachten, insbesondere kombinierten Zugriff auf Funktionen des Transfer-Dienstmoduls (24) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Transfer-Grundmodul (25) aus zugreifbar ist.

28. System (1) nach einem der Ansprüche 24 bis 27,
wobei das Transfer-Framework (22) weiter umfasst:
als Komponente einer der Serviceschicht (3) streng hierarchisch untergeordneten Web-Serviceschicht (6) Remote-Transfer-Dienste (33), die Funktionen zum Übertragen von Daten zwischen verschiedenen Knoten eines medizinischen Netzwerkes enthalten, wobei auf die Remote-Transfer-Dienste (33) von dem Dateizugangsmodul (10) aus zugreifbar ist.

29. System (1) nach einem der Ansprüche 24 bis 28,
wobei das Transfer-Framework (22) weiter umfasst:
als Komponente der Toolkit-Schicht (4) mindestens ein Transfer-Baukastenmodul (29), das einer Inhaltsseite (30) der Programmierumgebung (7) zuordenbar ist.

30. System (1) nach den Ansprüchen 16 und 27,
wobei ausgehend von dem Standard-Datenmanagement-Zugriffsmodul (14) eine Zugriffsmöglichkeit sowohl auf das Datenmangement-Dienstmodul (13) als auch auf das Transfer-Dienstmodul (24) vorgesehen ist.

31. System (1) nach einem der Ansprüche 1 bis 30, mit einem Aufgabenlisten-Framework (34) umfassend:
- als Komponenten der Serviceschicht (3)
- ein Dateizugangsmodul (10), das fundamentale Funktionen zum Lesen, Schreiben und Löschen von Daten sowie zum Erzeugen von Dateien in einem Dateisystem enthält,
- ein dem Dateizugangsmodul (10) übergeordnetes Arbeitsbereichsmodul (12), das Funktionen zur plattformunabhängigen lokalen Zwischenspeicherung von Daten unter Zugriff auf das Dateizugangsmodul (10) enthält sowie
- ein dem Arbeitsbereichsmodul (12) übergeordnetes Aufgabenlisten-Dienstmodul (35), das Funktionen zur Verknüpfung von Daten mit Aufgabenlisteneinträgen unter Rückgriff auf das Arbeitsbereichsmodul (12) umfasst,
- als Komponente der Toolkit-Schicht (4) ein Aufgabenlisten-Grundmodul (36), das Funktionen zum Zugriff auf das Aufgabenlisten-Dienstmodul (35) im Rahmen einer Applikation enthält.

32. System (1) nach Anspruch 31,
wobei das Aufgabenlisten-Framework (34) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Arbeitsbereichsmodul (12) aus zugreifbar ist.

33. System (1) nach Anspruch 31 oder 32,
wobei das Aufgabenlisten-Framework (34) weiter umfasst:
als Komponente der Serviceschicht (3)
- ein dem Dateizugangsmodul (10) und gegebenenfalls dem Informations-Modell-Abbildungsmodul (11) übergeordnetes Datenmanagement-Dienstmodul (13), das Funktionen zur Datenauswahl sowie zur Dateizugangsregelung unter Zugriff auf das Dateizugangsmodul (10) und gegebenenfalls auf das Informations-Modell-Abbildungsmodul (11) enthält,
- ein dem Datenmanagement-Dienstmodul (13) übergeordnetes Standard-Datenmanagement-Zugriffsmodul (14), das Funktionen zum vereinfachten, insbesondere kombinierten Zugriff auf Funktionen des Datenmanagement-Dienstmoduls (13) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Aufgabenlisten-Grundmodul (36) aus zugreifbar ist.

34. System (1) nach einem der Ansprüche 31 bis 33,
wobei das Aufgabenlisten-Framework (34) weiter umfasst:
als Komponente der Serviceschicht (3)
- ein dem Dateizugangsmodul (10) übergeordnetes Pipeline-Modul (23), das eine Anzahl von einzeln oder in beliebiger Kombination zuschaltbaren Filtern zur Bearbeitung von Bilddaten definiert, sowie
- ein dem Dateizugangsmodul (10) und dem Pipeline-Modul (23) übergeordnetes Transfer-Dienstmodul (24), das Funktionen zur Übertragung von Daten zwischen zwei Dateisystemen unter Zugriff auf das Dateizugangsmodul (10) und/oder gegebenenfalls das Informations-Modell-Abbildungsmodul (11) und/oder das Pipeline-Modul (23) enthält,
- ein dem Transfer-Dienstmodul (24) übergeordnetes Standard-Datenmanagement-Zugriffsmodul (14), das Funktionen zum vereinfachten, insbesondere kombinierten Zugriff auf Funktionen des Transfer-Dienstmoduls (24) sowie des Dateizugangsmoduls (10) und/oder gegebenenfalls des Informations-Modell-Abbildungsmoduls (11) enthält, wobei auf das Standard-Datenmanagement-Zugriffsmodul (14) seinerseits von dem Aufgabenlisten-Grundmodul (36) aus zugreifbar ist.

35. System (1) nach einem der Ansprüche 31 bis 34,
wobei das Aufgabenlisten-Framework (34) weiter umfasst:
als Komponente einer der Serviceschicht (3) streng hierarchisch untergeordneten Web-Serviceschicht (6)
- Remote-Datenmanagement-Dienste (17), die Funktionen zum Lesen, Schreiben und Löschen von Daten sowie zum Erzeugen von Dateien auf einem entfernten Knoten eines medizinischen Netzwerkes enthalten, und/oder
- Remote-Transfer-Dienste (33), die Funktionen zum Übertragen von Daten zwischen verschiedenen Knoten eines medizinischen Netzwerkes enthalten,
wobei auf die Remote-Datenmanagement-Dienste (17) bzw. Remote-Transfer-Dienste (33) von dem Dateizugangsmodul (10) aus zugreifbar ist.

36. System (1) nach einem der Ansprüche 31 bis 35,
wobei das Aufgabenlisten-Framework (34) weiter umfasst:
als Komponente einer der Serviceschicht (3) streng hierarchisch untergeordneten Web-Serviceschicht (6) Remote-Aufgabenlisten-Dienste (44), die die Funktionen zur netzwerküberspannenden Koordination von Aufgabenlisten enthalten, wobei auf die Remote-Aufgabenlisten-Dienste (44) direkt von dem Aufgabenlisten-Grundmodul (36) aus zugreifbar ist.

37. System (1) nach einem der Ansprüche 31 bis 36,
wobei das Aufgabenlisten-Framework (34) weiter umfasst:
als Komponente der Toolkit-Schicht (4) mindestens ein Aufgabenlisten-Baukastenmodul (40), das einer Inhaltsseite (41) der Programmierumgebung (7) zuordenbar ist, und das Funktionen zum Anzeigen, Erstellen und Bearbeiten von Aufgabenlisten unter Zugriff auf das Aufgabenlisten-Dienstmodul (35) und/oder gegebenenfalls das Standard-Datenmanagement-Modul (14) und/oder gegebenenfalls die Remote-Datenmanagement-Dienste (17) umfasst.

38. System (1) nach einem der Ansprüche 1 bis 37, mit einem Bildbearbeitungs-Framework (45) umfassend:
- als Komponenten der Serviceschicht (3)
- ein Segmentierungsmodul (46), das Funktionen zur Segmentierung von medizinischen Bilddaten enthält,
- ein dem Segmentierungsmodul (46) übergeordnetes Pipeline-Modul (23), das eine Anzahl von einzeln oder in beliebiger Kombination zuschaltbaren Filtern zur Bearbeitung von Bilddaten definiert, wobei von dem Pipeline-Modul (23) aus auf das Segmentierungsmodul (46) zugreifbar ist,
- ein dem Pipeline-Modul (23) nebengeordnetes Graphik/Anzeige-Modul (47), das Funktionen zur Handhabung und Anzeige von geschichteten Bildsegmentinhalten enthält, wobei von dem Graphik/Anzeige-Modul (47) aus auf das Segmentierungsmodul (46) zugreifbar ist, sowie
- ein dem Pipeline-Modul (23) und dem Graphik/Anzeige-Modul (47) übergeordnetes Bildbearbeitungs-Dienstmodul (48), das Funktionen zur Anzeige medizinischer Bilddaten unter Zugriff auf das Pipeline-Modul (23) und/oder das Anzeige/Graphik-Modul (47) und/oder das Segmentierungsmodul (46) umfasst,
- als Komponente der Toolkit-Schicht (4) ein Bildanzeige-Grundmodul (49), das Funktionen zum Zugriff auf das Bildbearbeitungs-Dienstmodul (48) im Rahmen einer Applikation enthält.

39. System (1) nach Anspruch 38,
wobei das Bildbearbeitungs-Framework (45) weiter umfasst:
als Komponente der Serviceschicht (3)
- ein Dateizugangsmodul (10), das fundamentale Funktionen zum Lesen, Schreiben und Löschen von Daten sowie zum Erzeugen von Dateien in einem Dateisystem enthält,
- ein dem Dateizugangsmodul (10) übergeordnetes Arbeitsbereichsmodul (12), das Funktionen zur plattformunabhängigen lokalen Zwischenspeicherung von Daten unter Zugriff auf das Dateizugangsmodul (10) enthält, wobei auf das Arbeitsbereichsmodul (12) von dem Pipeline-Modul (23) und dem Bildbearbeitungs-Dienstmodul (48) aus zugreifbar ist.

40. System (1) nach Anspruch 39,
wobei das Bildbearbeitungs-Framework (45) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Arbeitsbereichsmodul (12) aus zugreifbar ist.

41. System (1) nach Anspruch 39 oder 40,
wobei das Bildbearbeitungs-Framework (45) weiter umfasst:
als Komponente einer der Serviceschicht (3) streng hierarchisch untergeordneten Web-Serviceschicht (6)
- Remote-Datenmanagement-Dienste (17), die Funktionen zum Lesen, Schreiben und Löschen von Daten sowie zum Erzeugen von Dateien auf einem entfernten Knoten eines medizinischen Netzwerkes enthalten, und/oder
- Remote-Transfer-Dienste (33), die Funktionen zum Übertragen von Daten zwischen verschiedenen Knoten eines medizinischen Netzwerkes enthalten,
wobei auf die Remote-Datenmanagement-Dienste (17) bzw. Remote-Transfer-Dienste (33) von dem Dateizugangsmodul (10) aus zugreifbar ist.

42. System (1) nach einem der Ansprüche 38 bis 41,
wobei das Bildbearbeitungs-Framework (45) weiter umfasst:
als Komponente einer der Serviceschicht (3) streng hierarchisch untergeordneten Web-Serviceschicht (6) Remote-Display-Dienste (57), die Funktionen zur Anzeige von geschichteten Bildsegmentinhalten auf einem entfernten Knoten eines medizinischen Netzwerkes umfassen,
wobei auf die Remote-Display-Dienste (57) von dem Graphik/Anzeige-Modul (47) aus zugreifbar ist.

43. System (1) nach einem der Ansprüche 38 bis 42,
wobei im Rahmen des Bildbearbeitungs-Framework (45) als weitere Komponente der Toolkit-Schicht (4) mindestens ein Bildbearbeitungs-Baukastenmodul (53) vorgesehen ist, das einer Inhaltsseite (54) der Programmierumgebung (7) zuordenbar ist.

44. System (1) nach Anspruch einem der Ansprüche 39 oder 43,
wobei das Bildbearbeitungs-Framework (45) weiter umfasst:
als Komponente der Serviceschicht (3)
- ein dem Dateizugangsmodul (10) und gegebenenfalls dem Informations-Modell-Abbildungsmodul (11) übergeordnetes Datenmanagement-Dienstmodul (13), das Funktionen zur Datenauswahl sowie zur Dateizugangsregelung unter Zugriff auf das Dateizugangsmodul (10) und gegebenenfalls auf das Informations-Modell-Abbildungsmodul (11) enthält,
- ein dem Datenmanagement-Dienstmodul (13) übergeordnetes Standard-Datenmanagement-Zugriffsmodul (14), das Funktionen zum vereinfachten, insbesondere kombinierten Zugriff auf Funktionen des Datenmanagement-Dienstmoduls (13) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Bildbearbeitungs-Grundmodul (49) aus zugreifbar ist.

45. System (1) nach einem der Ansprüche 39 bis 44,
wobei das Bildbearbeitungs-Framework (45) weiter umfasst:
als Komponente der Serviceschicht (3)
- ein dem Pipeline-Modul (23) übergeordnetes Transfer-Dienstmodul (24), das Funktionen zur Übertragung von Daten zwischen zwei Knoten eines medizinischen Netzwerkes oder zwei Dateisystemen unter Zugriff auf das das Pipeline-Modul (23) und/oder das Dateizugangsmodul (10) und/oder gegebenenfalls das Informations-Modell-Abbildungsmodul (11) enthält,
- ein dem Transfer-Dienstmodul (24) übergeordnetes Standard-Datenmanagement-Zugriffsmodul (14), das Funktionen zum vereinfachten, insbesondere kombinierten Zugriff auf Funktionen des Transfer-Dienstmoduls (24) sowie des Dateizugangsmoduls (10) und/oder des Informations-Modell-Abbildungsmoduls (11) enthält, wobei auf das Standard-Datenmanagement-Zugriffsmodul (14) seinerseits von dem Bildbearbeitungs-Grundmodul (49) aus zugreifbar ist.

46. System (1) nach einem der Ansprüche 1 bis 45, mit einem Volumenbearbeitungs-Framework (58) umfassend:
- als Komponenten der Serviceschicht (3)
- ein Dateizugangsmodul (10), das fundamentale Funktionen zum Lesen, Schreiben und Löschen von Daten sowie zum Erzeugen von Dateien in einem Dateisystem enthält,
- ein dem Dateizugangsmodul (10) übergeordnetes Arbeitsbereichsmodul (12), das Funktionen zur plattformunabhängigen lokalen Zwischenspeicherung von Daten unter Zugriff auf das Dateizugangsmodul (10) enthält,
- ein Segmentierungsmodul (46), das Funktionen zur Segmentierung von medizinischen Bilddaten enthält,
- ein dem Segmentierungs-Modul (46) übergeordnetes Graphik/Anzeige-Modul (47), dass Funktionen zur Handhabung und Anzeige von geschichteten Bildsegmentinhalten enthält, wobei von dem Graphik/Anzeige-Modul (47) aus auf das Segmentierungsmodul (46) zugreifbar ist, sowie
- ein dem Graphik/Anzeige-Modul (47) übergeordnetes Volumenbearbeitungs-Dienstmodul (60), das Funktionen zur Bearbeitung und Anzeige von medizinischen Volumendaten unter Zugriff auf das Anzeige/Graphik-Modul (47) und/oder das Arbeitsbereichsmodul (12) umfasst,
- als Komponente der Toolkit-Schicht (4) ein Bildanzeige-Grundmodul (49), das Funktionen zum Zugriff auf das Volumenbearbeitungs-Dienstmodul (60) im Rahmen einer Applikation enthält.

47. System (1) nach Anspruch 46,
wobei das Volumenbearbeitungs-Framework (58) weiter umfasst:
als Komponente der Serviceschicht (3) ein 3D-Bildsynthese-Modul (59), das Funktionen zur Erstellung räumlicher Bilder anhand von 3D-Szenen umfasst, und auf das von dem Volumenbearbeitungs-Dienstmodul (60) aus zugreifbar ist.

48. System (1) nach Anspruch 47,
wobei das Volumenbearbeitungs-Framework (58) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Arbeitsbereichsmodul (13) aus zugreifbar ist.

49. System (1) nach einem der Ansprüche 46 bis 48,
wobei das Volumenbearbeitungs-Framework (58) weiter umfasst:
als Komponente einer der Serviceschicht (3) streng hierarchisch untergeordneten Web-Serviceschicht (6)
- Remote-Datenmanagement-Dienste (17), die Funktionen zum Lesen, Schreiben und Löschen von Daten sowie zum Erzeugen von Dateien auf einem entfernten Knoten eines medizinischen Netzwerkes enthalten, und/oder
- Remote-Transfer-Dienste (33), die Funktionen zum Übertragen von Daten zwischen verschiedenen Knoten eines medizinischen Netzwerkes enthalten,
wobei auf die Remote-Datenmanagement-Dienste (17) bzw. Remote-Transfer-Dienste (33) von dem Dateizugangsmodul (10) aus zugreifbar ist.

50. System (1) nach einem der Ansprüche 46 bis 49,
wobei das Volumenbearbeitungs-Framework (58) weiter umfasst:
als Komponente einer der Serviceschicht (3) streng hierarchisch untergeordneten Web-Serviceschicht (6) Remote-Volumenbearbeitungs-Dienste (68), die Funktionen zur Bearbeitung von Volumendaten auf einem entfernten Knoten eines medizinischen Netzwerkes umfassen, wobei auf die Remote-Volumenbearbeitungs-Dienste (68) von dem Volumenbearbeitungs-Dienstmodul (60) aus zugreifbar ist.

51. System (1) nach einem der Ansprüche 46 bis 50,
wobei das Volumenbearbeitungs-Framework (58) weiter umfasst:
als Komponente einer der Serviceschicht (3) streng hierarchisch untergeordneten Web-Serviceschicht (6) Remote-Display-Dienste (57), die Funktionen zur Anzeige von geschichteten Bildsegmentinhalten auf einem entfernten Knoten eines medizinischen Netzwerkes umfassen,
wobei auf die Remote-Display-Dienste (57) von dem Graphik/Anzeige-Modul (47) aus zugreifbar ist.

52. System (1) nach einem der Ansprüche 46 bis 51,
wobei im Rahmen des Volumenbearbeitungs-Framework (58) als weitere Komponente der Toolkit-Schicht (4) mindestens ein Volumenbearbeitungs-Baukastenmodul (64) vorgesehen ist, das einer Inhaltsseite (65) der Programmierumgebung (7) zuordenbar ist.

53. System (1) nach Anspruch einem der Ansprüche 46 bis 52,
wobei das Volumenbearbeitungs-Framework (58) weiter umfasst:
als Komponente der Serviceschicht (3)
- ein dem Dateizugangsmodul (10) und gegebenenfalls dem Informations-Modell-Abbildungsmodul (11) übergeordnetes Datenmanagement-Dienstmodul (13), das Funktionen zur Datenauswahl sowie zur Dateizugangsregelung unter Zugriff auf das Dateizugangsmodul (10) und gegebenenfalls auf das Informations-Modell-Abbildungsmodul (11) enthält,
- ein dem Datenmanagement-Dienstmodul (13) übergeordnetes Standard-Datenmanagement-Zugriffsmodul (14), das Funktionen zum vereinfachten, insbesondere kombinierten Zugriff auf Funktionen des Datenmanagement-Dienstmoduls (13) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Bildanzeige-Grundmodul (49) aus zugreifbar ist.

54. System (1) nach einem der Ansprüche 46 bis 53,
wobei das Volumenbearbeitungs-Framework (58) weiter umfasst:
als Komponente der Serviceschicht (3)
- ein dem Dateizugangsmodul (10) und gegebenenfalls dem Informations-Modell-Abbildungsmodul (11) übergeordnetes Transfer-Dienstmodul (13), das Funktionen zur Übertragung von Daten zwischen zwei Knoten eines medizinischen Netzwerkes oder zwei Dateisystemen unter Zugriff auf das Dateizugangsmodul (10) und/oder gegebenenfalls das Informations-Modell-Abbildungsmodul (11) enthält,
- ein dem Transfer-Dienstmodul (24) übergeordnetes Standard-Datenmanagement-Zugriffsmodul (14), das Funktionen zum vereinfachten, insbesondere kombinierten Zugriff auf Funktionen des Transfer-Dienstmoduls (13) sowie gegebenenfalls des Dateizugangsmoduls (10) und/oder des Informations-Modell-Abbildungsmoduls (11) enthält, wobei auf das Standard-Datenmanagement-Zugriffsmodul (14) seinerseits von dem Bildanzeige-Grundmodul (49) aus zugreifbar ist.

55. System (1) nach einem der Ansprüche 1 bis 54, mit einem Berichterstellungs-Framework (69) umfassend:
- als Komponenten der Serviceschicht (3)
- ein Dateizugangsmodul (10), das fundamentale Funktionen zum Lesen, Schreiben und Löschen von Daten sowie zum Erzeugen von Dateien in einem Dateisystem enthält,
- ein dem Dateizugangsmodul (10) übergeordnetes Arbeitsbereichsmodul (12), das Funktionen zur plattformunabhängigen lokalen Zwischenspeicherung von Daten unter Zugriff auf das Dateizugangsmodul (10) enthält,
- ein dem Arbeitsbereichsmodul (12) übergeordnetes Berichterstellungs-Dienstmodul (71), das Funktionen zum Erstellen und automatisierten Lesen von nach Maßgabe eines Datenstandards, insbesondere DICOM, strukturierten Berichten unter Zugriff auf das Arbeitsbereichsmodul (12) enthält,
- als Komponente der Toolkit-Schicht (4) ein Berichterstellungs-Grundmodul (72), das Funktionen zum Zugriff auf das Berichterstellungs-Dienstmodul (71) im Rahmen einer Applikation enthält.

56. System (1) nach Anspruch 55,
wobei das Berichterstellungs-Framework (69) weiter umfasst:
als Komponente der Serviceschicht (3) ein Terminologiemodul (70), das Textbausteine für die Berichterstellung zur Verfügung stellt, wobei auf das Terminologiemodul (70) von dem Berichterstellungs-Dienstmodul (71) aus zugreifbar ist.

57. System (1) nach Anspruch 55 oder 56,
wobei das Berichterstellungs-Framework (69) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Arbeitsbereichsmodul (12) aus zugreifbar ist.

58. System (1) nach einem der Ansprüche 55 bis 57,
wobei das Berichterstellungs-Framework (69) weiter umfasst:
als Komponente der Serviceschicht (3)
- ein dem Dateizugangsmodul (10) und gegebenenfalls dem Informations-Modell-Abbildungsmodul (11) übergeordnetes Datenmanagement-Dienstmodul (13), das Funktionen zur Datenauswahl sowie zur Dateizugangsregelung unter Zugriff auf das Dateizugangsmodul (10) und gegebenenfalls auf das Informations-Modell-Abbildungsmodul (11) enthält,
- ein dem Datenmanagement-Dienstmodul (13) übergeordnetes Standard-Datenmanagement-Zugriffsmodul (14), das Funktionen zum vereinfachten, insbesondere kombinierten Zugriff auf Funktionen des Datenmanagement-Dienstmoduls (13) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Berichterstellungs-Grundmodul (72) aus zugreifbar ist.

59. System (1) nach einem der Ansprüche 55 bis 58,
wobei das Berichterstellungs-Framework (69) weiter umfasst:
als Komponente der Serviceschicht (3)
- ein dem Arbeitsbereichsmodul (12) übergeordnetes Pipeline-Modul (23), das eine Anzahl von einzeln oder in beliebiger Kombination zuschaltbaren Filtern zur Bearbeitung von Bilddaten definiert, wobei von dem Pipeline-Modul (23) aus auf das Arbeitsbereichs-Modul (12) zugreifbar ist,
- ein dem Pipeline-Modul (23) übergeordnetes Transfer-Dienstmodul (13), das Funktionen zur Übertragung von Daten zwischen zwei Knoten eines medizinischen Netzwerkes oder zwei Dateisystemen unter Zugriff auf das Dateizugangsmodul (10), das Pipeline-Modul (23) und/oder gegebenenfalls das Informations-Modell-Abbildungsmodul (11) enthält,
- ein dem Transfer-Dienstmodul (24) übergeordnetes Standard-Datenmanagement-Zugriffsmodul (14), das Funktionen zum vereinfachten, insbesondere kombinierten Zugriff auf Funktionen des Transfer-Dienstmoduls (24) sowie gegebenenfalls des Dateizugangsmoduls (10) und/oder des Informations-Modell-Abbildungsmoduls (11) enthält, wobei auf das Standard-Datenmanagement-Zugriffsmodul (14) seinerseits von dem Berichterstellungs-Grundmodul (72) aus zugreifbar ist.

60. System (1) nach einem der Ansprüche 56 bis 59,
wobei das Berichterstellungs-Framework (69) weiter umfasst:
als Komponente einer der Serviceschicht (3) streng hierarchisch untergeordneten Web-Serviceschicht (6) einen Zentral-Terminologie-Service (80), der von einem entfernten Knoten eines medizinischen Netzwerkes aus Textbausteine für die Berichterstellung zur Verfügung stellt, wobei auf den Zentral-Terminologie-Service (80) von dem Terminolgiemodul (70) aus zugreifbar ist.

61. System (1) nach einem der Ansprüche 1 bis 60, mit einem Bilderfassungs-Framework (81) umfassend:
- als Komponente der Serviceschicht (3)ein Bilderfassungs-Dienstmodul (82), das Funktionen zur Erfassung von Rohdaten unter Zugriff auf mindestens eine medizinische Bildquelle sowie zur Aufbereitung dieser Rohdaten zu medizinischen Bilddaten und zur Echtzeit-Anzeige und Archivierung von Rohdaten und/oder aufbereiteten Bilddaten enthält,
- als Komponente der Toolkit-Schicht (4) ein Bilderfassungs-Grundmodul (84), das Funktionen zum Zugriff auf das Bilderfassungs-Dienstmodul (82) im Rahmen einer Applikation enthält.

62. System (1) nach Anspruch 61,
wobei das Bilderfassungs-Framework (81) weiter umfasst:
als Komponente der Serviceschicht (3) ein Dateizugangsmodul (10), das fundamentale Funktionen zum Lesen, Schreiben und Löschen von Daten sowie zum Erzeugen von Dateien in einem Dateisystem enthält, wobei auf das Dateizugangsmodul (10) von dem Bilderfassungs-Dienstmodul (82) aus zugreifbar ist.

63. System (1) nach Anspruch 61 oder 62,
wobei das Bilderfassungs-Framework (81) weiter umfasst:
als Komponente der Serviceschicht (3) ein dem Dateizugangsmodul (10) übergeordnetes Arbeitsbereichsmodul (12), das Funktionen zur plattformunabhängigen lokalen Zwischenspeicherung von Daten unter Zugriff auf das Dateizugangsmodul (10) enthält, wobei auf das Dateizugangsmodul (10) von dem Bilderfassungs-Dienstmodul (82) aus zugreifbar ist.

64. System (1) nach einem der Ansprüche 61 bis 63,
wobei das Bilderfassungs-Framework (81) weiter umfasst:
als Komponenten der Serviceschicht (3)
- ein Segmentierungsmodul (46), das Funktionen zur Segmentierung von medizinischen Bilddaten enthält,
- ein dem Segmentierungsmodul (46) übergeordnetes Pipeline-Modul (23), das eine Anzahl von einzeln oder in beliebiger Kombination zuschaltbaren Filtern zur Bearbeitung von Bilddaten definiert, wobei von dem Pipeline-Modul (23) aus auf das Segmentierungsmodul (46) und gegebenenfalls das Arbeitsbereichsmodul (12) zugreifbar ist,
- ein dem Pipeline-Modul (23) nebengeordnetes Graphik/Anzeige-Modul (47), das Funktionen zur Handhabung und Anzeige von geschichteten Bildsegmentinhalten enthält, wobei von dem Graphik/Anzeige-Modul (47) aus auf das Segmentierungsmodul (46) zugreifbar ist, sowie
- ein dem Pipeline-Modul (23) und dem Graphik/Anzeige-Modul (47) übergeordnetes, und dem Bilderfassungs-Dienstmodul neben- oder untergeordnetes Bildbearbeitungs-Dienstmodul (48), das Funktionen zur Anzeige medizinischer Bilddaten unter Zugriff auf das Pipeline-Modul (23) und/oder das Anzeige/Graphik-Modul (47) und/oder das Segmentierungsmodul (46) umfasst,
wobei von dem Bilderfassungs-Dienstmodul (82) aus auf das Segmentierungsmodul (46) und das Graphik/Anzeige-Modul (47) zugreifbar ist, wobei von dem Bildbearbeitungs-Dienstmodul (48) aus auf das Arbeitsbereichsmodul (12) zugreifbar ist, und wobei auf das Bildbearbeitungs-Dienstmodul (48) seinerseits von dem Bilderfassungs-Grundmodul (84) aus zugreifbar ist.

65. System (1) nach einem der Ansprüche 61 bis 64,
wobei das Bilderfassungs-Framework (81) weiter umfasst:
als Komponente der Serviceschicht (3) ein dem Dateizugangsmodul (10) nebengeordnetes Informations-Modell-Abbildungsmodul (11), das Funktionen zur Erzeugung einer nach einem Datenstandard, insbesondere DICOM, eindeutigen Referenz auf eine Datei anhand von dieser Datei zugeordneten Meta-Daten sowie zur Analyse und Gruppierung solcher Referenzen enthält, wobei auf das Informations-Modell-Abbildungsmodul (11) von dem Arbeitsbereichsmodul (12) und dem Bilderfassungs-Dienstmodul (82) aus zugreifbar ist.

66. System (1) nach Anspruch 65,
wobei das Bilderfassungs-Framework (81) weiter umfasst:
als Komponente der Serviceschicht (3) ein 3D-Bildsynthese-Modul (59), das Funktionen zur Erstellung räumlicher Bilder anhand von 3D-Szenen umfasst, wobei von dem Bilderfassungs-Dienstmodul (82) aus auf das 3D-Bildsynthese-Modul (59) zugreifbar ist.

67. System (1) nach einem der Ansprüche 62 bis 66, weiter umfassend als Komponenten der Serviceschicht (3)
- ein dem Dateizugangsmodul (10) und gegebenenfalls dem Informations-Modell-Abbildungsmodul (11) übergeordnetes Datenmanagement-Dienstmodul (13), das Funktionen zur Datenauswahl sowie zur Dateizugangsregelung unter Zugriff auf das Dateizugangsmodul (10) und gegebenenfalls auf das Informations-Modell-Abbildungsmodul (11) enthält,
- ein dem Datenmanagement-Dienstmodul (13) übergeordnetes Standard-Datenmanagement-Zugriffsmodul (14), das Funktionen zum vereinfachten, insbesondere kombinierten Zugriff auf Funktionen des Datenmanagement-Dienstmoduls (13) sowie des Dateizugangsmoduls (10) und gegebenenfalls des Informations-Modell-Abbildungsmoduls (11) enthält, wobei auf das Standard-Datenmanagement-Zugriffsmodul (14) seinerseits von dem Bilderfassungs-Grundmodul (84) aus zugreifbar ist.

68. System (1) nach einem der Ansprüche 62 bis 67,
wobei das Bilderfassungs-Framework (81) weiter umfasst:
als Komponente der Serviceschicht (3)
- ein dem Dateizugangsmodul (10) und gegebenenfalls dem Informations-Modell-Abbildungsmodul (11) übergeordnetes Transfer-Dienstmodul (24), das Funktionen zur Übertragung von Daten zwischen zwei Knoten eines medizinischen Netzwerkes oder zwei Dateisystemen unter Zugriff auf das Dateizugangsmodul (10) und/oder gegebenenfalls das Informations-Modell-Abbildungsmodul (11) und/oder gegebenenfalls das Pipeline-Modul (10) enthält,
- ein dem Transfer-Dienstmodul (24) übergeordnetes Standard-Datenmanagement-Zugriffsmodul (14), das Funktionen zum vereinfachten, insbesondere kombinierten Zugriff auf Funktionen des Transfer-Dienstmoduls (13) sowie gegebenenfalls des Dateizugangsmoduls (10) und/oder des Informations-Modell-Abbildungsmoduls (11) enthält, wobei auf das Standard-Datenmanagement-Zugriffsmodul (14) seinerseits von dem Bilderfassungs-Grundmodul (84) aus zugreifbar ist.

69. System (1) nach Anspruch 62 oder 68,
wobei das Bilderfassungs-Framework (81) weiter umfasst:
als Komponente einer der Serviceschicht (3) streng hierarchisch untergeordneten Web-Serviceschicht (6)
- Remote-Datenmanagement-Dienste (17), die Funktionen zum Lesen, Schreiben und Löschen von Daten sowie zum Erzeugen von Dateien auf einem entfernten Knoten eines medizinischen Netzwerkes enthalten, und/oder
- Remote-Transfer-Dienste (33), die Funktionen zum Übertragen von Daten zwischen verschiedenen Knoten eines medizinischen Netzwerkes enthalten,
wobei auf die Remote-Datenmanagement-Dienste (17) bzw. Remote-Transfer-Dienste (33) von dem Dateizugangsmodul (10) aus zugreifbar ist.

70. System (1) nach einem der Ansprüche 64 bis 69,
wobei das Bilderfassungs-Framework (81) weiter umfasst:
als Komponente einer der Serviceschicht (3) streng hierarchisch untergeordneten Web-Serviceschicht (6) Remote-Display-Dienste (57), die Funktionen zur Anzeige von geschichteten Bildsegmentinhalten auf einem entfernten Knoten eines medizinischen Netzwerkes umfassen,
wobei auf die Remote-Display-Dienste (57) von dem Graphik/Anzeige-Modul (47) aus zugreifbar ist.

71. System (1) nach einem der Ansprüche 61 bis 70,
wobei das Bilderfassungs-Framework (81) weiter umfasst:
als Komponente einer der Serviceschicht (3) streng hierarchisch untergeordneten Web-Serviceschicht (6) Remote-Bilderfassungs-Dienste (83), die Funktionen Erfassung von Rohdaten unter Zugriff auf mindestens eine medizinische Bildquelle sowie zur Aufbereitung dieser Rohdaten auf einem entfernten Knoten eines medizinischen Netzwerkes umfasst, wobei auf die Remote-Bilderfassungs-Dienste (83) von dem Bilderfassungs-Dienstmodul (82) aus zugreifbar ist.

72. System (1) nach einem der Ansprüche 61 bis 71,
wobei das Bilderfassung-Dienstmodul (13) ein Sub-System von Funktionen zur Kommunikation mit mindestens einer Bildquelle enthält.

73. System (1) nach einem der Ansprüche 61 bis 72,
wobei das Bilderfassung-Dienstmodul (13) ein Sub-System von Funktionen zur Anpassung von bildquellenspezifischen Rohdaten und/oder zugeordneten Meta-Daten an einen gemeinsamen Datenstandard, insbesondere DICOM, enthält.
